# EUROPEAN PATENT SPECIFICATION

(11) **EP 1 003 853 B1**
(45) Date of publication and mention of the grant of the patent: **30.11.2005**
(21) Application number: 98940471.0
(22) Date of filing: 03.09.1998
(51) Int. Cl.: C12N 15/10, C12N 11/14, C07K 17/00, C12Q 1/68, C12P 21/00, C12N 15/12

(54) **METHODS FOR PROTEIN SCREENING**
METHODEN ZUR DURCHFORSTUNG (SCREENEN) VON PROTEINEN
PROCEDES D'ANALYSE DE PROTEINES

(30) Priority: 03.09.1997 GB 9718552; 18.09.1997 GB 9719834; 24.09.1997 GB 9720184; 29.09.1997 GB 9720522; 29.09.1997 GB 9720525; 29.09.1997 GB 9720523; 29.09.1997 GB 9720524; 30.12.1997 US 70063 P; 30.12.1997 US 70062 P; 30.12.1997 US 70037 P; 30.12.1997 US 70050 P; 22.01.1998 GB 9801255; 25.02.1998 GB 9803828; 14.04.1998 GB 9807760; 23.05.1998 GB 9811130
(43) Date of publication of application: 31.05.2000
(73) Proprietor: Biovation Limited, Aberdeen AB10 1DQ (GB)
(72) Inventor: CARR, Francis c/o Biovation Limited Crombie Lodge, Aberdeen, AB 22 8 GU (GB); CARTER, Graham c/o Biovation Ltd., Crombie Lodge, Aberdeen, AB22 8GU (GB); HAMILTON, Anita c/o Biovation Ltd., Crombie Lodge, Aberdeen, AB22 8GU (GB); ADAIR, Fiona, Ellon Aberdeenshire AB41 7NH (GB); WILLIAMS, Steven, Insch Aberdeenshire AB52 6QD (GB)
(74) Representative: Benz, Jürgen
(86) International application number: PCT/GB1998/002649
(87) International publication number: WO 1999/011777

(56) References cited:
- WO-A-91/05058
- WO-A-92/02536
- WO-A-92/18645
- WO-A-93/03172
- WO-A-95/11922
- WO-A-95/34648
- WO-A-98/48008
- DE-C- 19 646 372
- HANES J & PL CKTHUN A: "In vitro selection and evolution of functional proteins by using ribosome display" PROCEEDINGS OF THE NATIONAL ACADEMY OF SCIENCES OF USA, no. 94, page 4937 4937 XP002077149 cited in the application
- MATTHEAKIS L C ET AL: "AN IN VITRO POLYSOME DISPLAY SYSTEM FOR IDENTIFYING LIGANDS FROM VERY LARGE PEPTIDE LIBRARIES" PROCEEDINGS OF THE NATIONAL ACADEMY OF SCIENCES OF USA, vol. 91, September 1994, pages 9022-9026, XP002043737 cited in the application
- S. JUNG AND A. PLÜCKTHUN: "Improving in vivo folding and stability of a single-chain-Fv antibody fragmnet by loop grafting" PROTEIN ENGINEERING, vol. 10, no. 8, August 1997, pages 959-966, XP002085709 IRL PRESS LIMITED OXFORD, ENGLAND

## Description

The present invention relates to methods and compositions for the screening of proteins and polypeptides. In particular, the invention relates to methods involving the generation of gene libraries and the synthesis of individual proteins or polypeptides which can be screened in various ways. Particular embodiments include the use of so-called "ribosome display" protein or polypeptide arrays. The invention therefore provides for novel practical applications of displayed protein or polypeptide arrays in screening for biologically active polypeptides, for proteins which bind to receptors and for interactions of proteins with other molecules.

From the ongoing initiative to sequence the human genome has developed a major initiative to find polymorphisms or mutations in human genes which might have causal relationships with human diseases and, in addition, to alterations in the expression of genes which might relate to disease. This has given rise to many high-throughput screening technologies in order to find such polymorphisms, mutations or alterations in gene expression. In addition, such analyses are now being undertaken to analyse human responses to certain treatments and therefore to predict responsiveness to these treatments. Commonly, polymorphisms, mutations and gene expression changes are reflected in proteins produced by these genes and it is the actions of these proteins which can directly determine biological outcomes such as development of a disease or response to a treatment. In addition, most eukaryotic proteins are modified post-translationally and such modifications cannot be analysed at the gene level. It therefore follows that analysis of the screening for proteins and protein modifications is more likely to give a precise relationship of changes with disease compared to analysis of DNA or RNA.

The analysis of protein associations with disease has, to date, been dominated by 2D (two-dimensional) protein gel analysis from human tissue or cells. For this technology, cellular proteins are usually separated on the basis of charge in one dimension and on the basis of size in the other dimension. Proteins can either be identified with reference to the electophoresis migration pattern of a known protein or by elution of the protein from the electrophoretically separated spot and analysis by methods such as mass spectrometry and nuclear magnetic resonance. However, limitations of the 2D protein gel method include the limited resolution and detection of proteins from a cell (typically only 5000 cellular proteins are clearly detected), the limitation to identification of separated proteins (for example, mass spectrometry usually requires 100fmoles or more of protein for identification), and the specialist nature of the technique. In addition, the analysis of post-translational modification of proteins by 2D gel analysis is limited and protein-protein binding interactions cannot be detected by this method.

A large number of cellular processes are controlled by the transient interaction between a modifying enzyme and its protein substrate. Such interactions are difficult to detect by conventional methods. Protein modifying enzymes such as kinases, phosphatases, transferases, proteases etc. control all manner of fundamental cellular processes (Pawson, Nature, 373 (1995), pp573) and have also been shown to be involved in disease pathways. These transient protein/protein interactions can result in a number of different effects some of which can be measured. The kinetic properties of a protein can be altered resulting in altered binding of substrates (Prelich et al., Nature 326, (1989) pp 517) or altered catalysis (Porpaczy et al., Biochim. Biophys. Acta, 749 (1983), pp 172). Protein/protein interactions can cause the formation of a new binding site e.g. an ATP binding site is formed by the interaction of the a and b subunits of the E. coli ATPase (Weber et al., J. Biol.Chem., 268, (1993), pp 6241). Substrate specificity of a protein can be altered by protein/protein interactions as exemplified by the interaction of different transcription factors with RNA polymerase directing the polymerase to specific promoters (White & Jackson, Trends Genet., 8, (1988), pp 284). Alternatively protein/protein interactions can cause inactivation, for example when a protein interacts with an inhibitor (Vincent & Lazdunski, Biochemistry, 11, (1972), pp2967).

There are methods disclosed in the art which rely on the generation of proteins or polypeptides from gene libraries. In vitro methods for the selection of a polypeptide potentially offer advantages over in vivo methods by eliminating the need for uptake of genes into cells and by controlling the environment for mRNA and protein production. In vitro transcription and translation reactions have been used for many years as a means of generating polypeptides directly from DNA and it has been shown that specific mRNAs can be enriched by the immunoprecipitation of polysomes (e.g. Payvar, F. and Schimke, R.T., Eur. J. Biochem., vol 101 (1979) p1844-1848) using antibodies to select the specific polypeptides. This so-called "ribosome display" technique has more recently been adapted for the selection of Peptides (Mattheakis. L. et al, PNAS 91: 9022, 1994 and PCT95/11922) and proteins (Kawasaki, G., PCT91/05058 and Hanes & Pluckthun, PNAS, vol 94 [10]:493 7, 1997).
Hanes & Pluckthun disclose an in vitro method for screening of correctly folded complete proteins and its encoding polynucleotides, by in vitro translation of the polynucleotide into protein, wherein the ribosome-bound protein is immobilised to a solid support by interaction to an ligand/antigen, and the interacting protein is selected and the mRNA is reverse transcribed into DNA.
However, one major disadvantage with such methods is that the proteins or polypeptides are generated as a "pool" which is then screened.
Thus, there is a need for screening methods which allow for rapid identification of individual proteins or polypeptides, and which in turn will also allow for identification of genes coding for such proteins or polypeptides.

Thus, in a first aspect, the present invention provides a method of screening proteins or polypeptides which comprises forming a gene library and synthesising individual proteins or polypeptides which can then be screened. In the context of the present invention, the term "individual proteins or polypeptides" means that the proteins or polypeptides expressed from the gene library are individually identifiable, rather than forming part of a pool. Thus, for instance, the proteins or polypeptides can be expresed as an array wherein each protein or polypeptide occupies a distinct point or area within the array. For example, a gene library may be generated in the form of colonies or plaques, which in turn can be picked off individually and arranged in an array.
Expression of the genes will ion turn result in an array of proteins or polypeptides in an array format, each occupying a distinct point or area.

The proteins or polypeptides generated from the gene libraries may be termed "synthetic proteins or polypeptides" produced by in vitro methods such as in vitro transcription and translation, ribosome display and phage display. Thus, they may be distinguished on that basis from "natural proteins" derived directly from tissue or cell extracts.

Thus, the methods described herein allow for screening of proteins and polypeptides and post-translational modifications using gene libraries as the starting point for synthesis of "synthetic" proteins or polypeptides. In general this is achieved by the generation of arrays of proteins or polypeptides as a means for screening them. In particular, the arrays are generated by in vitro transcription and translation methods.

Protein "Arrays" - in this embodiment of the present invention, there are provided novel methods for screening proteins and polypeptides and post-translational modifications using gene libraries as the starting point for synthesis of synthetic proteins. In this embodiment, these gene libraries provide the basis for segregating individual proteins or groups of proteins in order to detect polypeptides and to detect polypeptide modifications. The method principally avoids the use of protein gel electrophoresis. The invention provides methods for the high-throughput analysis of proteins in normal and disease tissues or cells principally to detect differences between normal and disease proteins and also for the analysis of protein changes in relation to drug and other disease treatments. The method also provides for high-throughput analysis of the binding or biological activity of libraries of proteins or polypeptides when exposed to living cells or tissues. Furthermore, the method also provides for the protein "fingerprinting" of individuals as an alternative to genetic fingerprinting in order to identify individuals and different tissues or cells. It is an important feature of the present invention that proteins are analysed either in pools or in arrays of individual proteins whereby, once modifications, binding or biological activity are detected using a variety of methods, the identity of the protein can be determined either directly or indirectly by either its location in a particular pool or its location at a particular position in an array. Equally, it is an important aspect of the present invention that the methods allow for the comparative detection of differences in proteins or protein modifications between different tissues or cells and therefore the methods constitute a screen for these differences.

The method is based primarily on the use of synthetic proteins produced by recombinant DNA methods, especially proteins produced from one or more genes by in vitro transcription and translation (IVTT). One preferred aspect of the present invention is the generation of synthetic proteins by IVTT with their subsequent immobilisation onto solid phases prior to screening for protein modification or for protein-protein binding interactions. It is an important aspect of this embodiment of the present invention that the generation of synthetic proteins from arrays of genes (such as cDNAs cloned into vectors designed to facilitate IVTT) is undertaken in such manner that synthetic proteins, once tested for protein modification, binding or biological activity can easily be identified by reference to the originating genes encoding these synthetic proteins whereby the gene sequence will reveal the identity of the protein. Such gene arrays are, for example, provided by picking individual cDNA clones or mixtures of clones into specific loci in an array (such as in the wells of a microtitre plate). Following IVTT (usually after amplification of the cDNAs by methods such as PCR), protein samples from each loci in the array can be taken and immobilised or dispensed into specific loci in another array, for example into alternative microtitre plates or onto a solid phase where samples are dispensed at individual loci on the solid phase. Where specific protein modifications or protein binding events are detected from protein samples in the array, then the specific gene encoding these proteins can be located and sequenced in order to identify the protein of interest.

It is also within the scope of the preferred embodiment present invention that the cDNA library may encode a set of variable molecules such as immunoglobulin variable regions (commonly as single-chain Fv regions, referred to as SCAs (single-chain antibodies)). Such variable molecules, when dispensed to specific loci in arrays, then have the ability to bind to other specific molecules (usually proteins) from cell or tissue samples. These cell or tissue molecules, by virtue of binding to the variable molecules in the array, then essentially provide for an ordered array of cell or tissue molecules. The presence of cell or tissue molecules in such arrays or modification of these molecules such as by phosphorylation or interaction with other proteins can then be addressed in the array. The identity of such cell or tissue molecules can subsequently be determined by several methods especially where the original variable molecules can be retrieved in isolation and used to retrieve the cell or tissue molecule(s) in isolation. The identity of such cell or tissue molecules can then be determined by classical biochemical means or, where these are proteins, by protein sequencing or by methods such as 2D gel migration properties. Alternatively, the identity of such proteinaceous cell or tissue molecules can be determined using a protein display system such as the ribosome system included in this patent whereby a cDNA library is used to generate a library of proteins linked to their genes (or RNA representation of the gene) such that, upon binding to a specific variable molecule, the identity of the binding protein can be determined by determining the nucleotide sequence of the associated gene.

As an alternative to the use of IVTT for generation of synthetic proteins in the formation of protein arrays, another aspect of the microarray embodiment of the invention is the use of "polypeptide display" methods for generating synthetic polypeptides with useful phenotypes from gene libraries encoding a large mixture of polypeptides whereby the corresponding genes can be recovered swiftly and easily. In vitro methods of polypeptide display have been developed which allow greater scope for the type of protein displayed than cell based display systems and in particular display of proteins associated with ribosomes ("ribosome display") which can provide proteins linked via the ribosome complex to corresponding mRNAs which can then be sequenced in order to reveal the identity of the protein. The present method can also incorporate other protein display methods such as phage display.

In one embodiment of the present method, protein modifications are screened by creation of a gene library which is then used to create synthetic proteins for example by IVTT to successively produce mRNA and protein from such vectors preferably under such conditions as to maximise yield of full-length synthetic proteins which have disassociated from mRNA. In order to avoid over-representation of proteins or polypeptides from abundant mRNA species, the gene library is normalised. Through the incorporation of modified amino acids such as biotinyl-lysine or the incorporation of an amino acid sequence tag encoded by the plasmid vector or introduced by PCR, or through chemical modification of the protein, the synthetic protein can next be immobilised onto a solid phase such as a magnetic bead via a biotin binding protein (e.g. streptavidin) or via a sequence tag binding protein such as an anti-tag antibody. In a screen for protein modification, through appropriate distribution of the starting genes, small pools of proteins or individual proteins are produced in arrays such as in microtitre wells and immobilised in these arrays.

Alternatively, the synthetic proteins might be produced in arrays as individual small pools of proteins or individual proteins and then transferred individually (either manually or by use of an automatic dispenser) to different loci on a flat solid phase such as on a glass "chip" whereby the proteins interact with functional moieties on the solid phase resulting in immobilisation. Next, these synthetic proteins are treated with extracts of tissues or cells whereby enzymes present in these tissues or cells can effect modifications to the synthetic proteins. Finally, such modifications are then be detected using specific detection systems such as fluorescently labelled anti-phoshotyrosine antibodies. If the signal from the detection system is compared from replicate arrays of synthetic proteins which are modified by different extracts of tissues or cells, then differences in these signals between replicate synthetic proteins will indicate differences in the protein modification activities from these different tissues or cells.

In another embodiment of the present method, protein-protein binding interactions are screened by creation of a gene library which is then used to create synthetic proteins such as by IVTT, preferably in such vectors and under such conditions as to maximise yield of full-length synthetic primary proteins which have disassociated from mRNA. Through the incorporation of modified amino acids or an amino acid sequence tag, or through chemical modification of the protein, or by use of a chemically reactive solid phase, the synthetic primary protein can be immobilised onto a solid phase either immediately after synthesis or following the interaction with secondary proteins to form any protein-protein interactions. Through appropriate distribution of the starting genes, as in screens for protein modification, small pools of primary proteins or individual proteins are produced in arrays such as in microtitre wells or on glass chips and immobilised in these arrays. Next, these synthetic primary proteins can optionally be treated with extracts of tissues or cells whereby enzymes present in these tissues or cells can effect modifications to the synthetic proteins. Next, one or more (including a library of) secondary proteins are then added in order to form any suitable binding interactions of appropriate secondary proteins with the primary proteins. Optionally, these secondary proteins may be synthetic and displayed on ribosomes or phage such that the identity of the secondary protein can subsequently be determined.

Alternatively, the secondary proteins could be natural proteins derived from tissues or cells. Protein-protein binding interactions between primary and secondary proteins can then be detected by a range of methods. For example, the primary protein can be immobilised onto a solid phase and the presence of a synthetic secondary protein determined via an amino acid sequence tag incorporated into the secondary protein with detection using specific detection systems such as fluorescently labelled anti-tag antibody. Alternatively, if the secondary protein is associated with its coding nucleic acid such as for ribosome or phage display, then the secondary protein can be detected by a PCR assay for the associated nucleic acid. Alternatively, binding of the secondary protein may be detected through the action of the secondary protein in blocking a site on the synthetic primary protein which is detected in the assay system and which becomes blocked if a secondary protein binds. Alternatively, if the secondary protein is a natural protein, it may be labelled directly after binding, for example by addition of a UV-activatable biotin molecule, and detected directly, or it may be laser vapourised and detected by MALDI (Matrix Assisted Laser Desorption Ionisation) and mass spectrometry. If the binding of secondary proteins to replicate arrays of synthetic primary proteins is compared betweendifferent extracts of tissues or cells, then differences in these signals between replicate synthetic proteins will indicate differences in the protein-protein binding activities from these different tissues or cells.

It will be obvious that by detecting a range of protein modifications and by detecting protein-protein binding with a common detection format such as by using fluorescent labelled antibodies to protein tags or epitopes, then simultaneous parallel screening for a number of different protein modifications could be undertaken in order to screen for differences between different tissues and cells.

In another embodiment of the present invention, protein-protein interactions are used as the basis for forming arrays of secondary proteins, especially natural tissue or cell derived proteins which normally would be very difficult to comprehensively array using standard technology. The primary proteins comprise a highly diverse set of proteins such as immunoglobulins which provide a diverse set of binding specificities for a set of secondary proteins such as natural proteins from tissue or cell extracts or synthetic proteins from a ribosome or phage displayed protein library. As an example, the primary proteins would derive from a Ribosome displayed single-chain antibody (SCA) variable region library formed by direct PCR amplification of immunoglobulin variable regions from mammalian B lymphocytes and cloning into suitable vectors for in vitro transcription. Members of the SCA library are arrayed either individually or in groups into microtitre wells or directly on a solid phase such as a glass chip and the SCA proteins are produced by IVTT and immobilised via a protein tag or by direct chemical coupling to a solid phase. Then a mixture of natural proteins from a tissue or cell extract or a mixture of synthetic proteins originating from a gene library (for example ribosome or phage displayed proteins) is added in bulk to the array of primary proteins such as SCAs whereby the binding specificity imparted by the primary proteins for the secondary proteins leads to arraying of the secondary proteins with either single secondary proteins or groups of secondary proteins bindign to individual SCAs. The achieved array of secondary proteins is compared between different extracts of tissues or cells, then differences in these signals between replicate synthetic proteins will indicate differences in the presence of proteins from these different tissues or cells. Alternatively, these proteins can also be tested for various modifications such a phosphorylation by using, for example, fluorescently labelled antibodies to detect these modifications.

It is a particular aspect of the present invention that proteins, either synthetic or natural, are distributed into arrays of single or multiple proteins in order to provide for comparisons of the presence or modification of these individual single or multiple protein members of the array between different tissue or cell extracts or to provide for analysis of the binding or biological activities of these protein members on live cells or tissue. Whilst arrays can readily be performed in microtitre plates by distributing individual members of groups of clones from a cDNA library in the individual wells of the plate, this manual (or robot-directed) arraying technology results in the physical barrier of the walls of the wells between different samples and this makes it difficult to add subsequent solutions such as wash solutions and libraries of secondary proteins quickly and accurately. In addition, the use of microtitre plates provides a limit to the density of arrayed proteins, such limit being dictated by the density of wells in the microtire plates available. It is therefore desirable to have methods which do not require arraying of proteins without physical barriers between the proteins.

In another embodiment of the invention where arrays of proteins are formed without physical barriers, the spatial distribution of proteins into arrays is achieved either by directly spatially immobilising individual synthetic proteins derived from a gene library by, for example, ribosome display or by spatially immobilising nucleic acids, especially synthetic DNA, to which nucleic acids associated with a synthetic protein library are annealed in order to indirectly spatially immobilise members of the protein library. For direct spatial immobilisation, either synthetic proteins derived from a tissue or cell gene library are immobilised or synthetic protein probes such as SCAs are immobilised such that a mixture of secondary proteins can then be added whereby these would be spatially distributed according to their binding specificities for individual protein probes. Thus, the invention provides for new types of protein chips (cfHutchens & Yip, Rapid Comms. Mass Spec. 7, (1993), pp576). Immobilisation of synthetic proteins on the chip is achieved by either covalent or non-covalent linkage. Particularly convenient is immobilisation using biotin moieties incorporated into the protein by addition of biotinyl lysine tRNA during the translation reaction. Having incorporated biotin, synthetic protein molecules can then readily be immobilised onto solid-phase streptavidin or solid-phase anti-biotin antibodies. Typically, this is facilitated by the use of a robotic dispenser which can aliquot protein samples produced by in vitro transcription and translation onto specific positions in a multiwell plate or onto a solid phase such as a glass chip. Such a robotic dispenser is particularly important for creating replicate arrays of synthetic proteins where identical proteins are immobilised at identical loci within the replicate solid phases. In relation to direct immobilisation using biotinyl lysine, free biotinyl lysine tRNA which has not incorporated into the synthetic protein can present difficulties in some samples whereby the free biotin groups block access to immobilised streptavidin molecules. Conveniently, this can be overcome by ensuring exhaustion of the biotinyl lysine tRNA in the translation reaction mixture (nascent proteins can then be completed by addition of unmodified lysine tRNA); alternative strategies are the use of solid phases with the number of biotin binding sites in excess of numbers of lysine-associated biotin molecules or a molecular sieve filtration step to remove small molecules such as biotinyl-lysine tRNA. Another strategy is to add into the translation reaction mixture, at the end of the translation period, a synthetic mRNA template encoding a lysine rich (poly)peptide which can then incorporate excess free biotinyl tRNAs. Such a template would also encode a peptide sequence (for example, a polyhistidine tail) which subsequently could be used to remove the lysine rich synthetic protein from the reaction mix (for example, using beads with antibodies against the polyhistidine tail). In such manner, the free biotinyl-lysine groups would be removed from the translation mixture prior to immobilisation of the synthetic proteins.

Non-covalent attachment of the proteins can also be facilitated by labelling the proteins for example with biotin using a commercially available reagent such as Sulfo-SBED (Pierce & Warriner, Chester, UK) which is then reacted with avidin. Covalent attachment of the proteins can also be achieved by activating the proteins with reactive species to facilitate cross-linking by any of the conventional ways, such as those described in OSullivan et al., (Anal. Biochem. 100 (1979),108). Attachment of proteins could also be facilitated by inclusion of specific amino acids sequences within the synthetic proteins such as terminal poly-histidine sequences which can then bind to either solid phase nickel chelates or anti-poly-histidine antibodies as a means for achieving imobilisation. Alternative methods for directly binding synthetic proteins would include binding of ribosome displayed proteins by immobilising the associated RNA using, for example, an RNA binding protein (such as HIV tat protein) or by annealing the RNA to synthetic DNA molecules on the solid phase.

For indirect immobilisation of proteins via nucleic acids, the associated mRNA in a protein-ribosome-mRNA complex may be produced with a nucleotide sequence tag originally encoded by the plasmid vector encoding the mRNA. The nucleotide sequence tag may be variable or randomised in the plasmid vector preparation, for example by producing the plasmid vector using a synthetic region encoded by a random oligonucleotide mixture (with appropriate ends which anneal to the vector) to place random a sequence tag at, for example, the 5 end of the mRNA. Following production of the protein-ribosome-mRNA complexes from the gene library, the mRNA sequence tags may then be annealed to an array of synthetic oligonucleotides individually positioned over a solid surface such as a glass slide (a "DNA biochip"). In this manner, individual proteins may finally be positioned at specific locations on the biochip. The spatially arrayed proteins may then be analysed for modification by tissue or cell extracts using, for example, fluorescent antibodies to screen for these modifications. In addition, the arrays may be used for identification of proteins which bind or modify the individual proteins on the biochip. The identity of the modified proteins or the proteins involved in protein-protein interactions may subsequently be determined from the known sequence of the immobilised oligonucleotide on the chip whereby this sequence is used to probe the plasmid library (for example, by PCR) in order to identify the specific gene associated with the complementary sequence tag within the plasmid library. The identity of the proteins may alternatively be achieved by PCR amplification of the mRNA sequence associated with the individual protein. This embodiment therefore relates to a protein array biochip with, in principle, individual proteins located at individual loci on the chip. Alternatively, the identity of the protein may be determined if the primary or secondary synthetic protein is produced with a random set of protein sequence tags which can be interrogated using, for example, a mixture of antibodies against those sequence tags. If, for example, the synthetic proteins are produced in such a manner that one terminus comprises either a random or semi-random stretch of amino acidseach of which can be detected by an antibody from a library of antibodies with appropriate labels, then the binding of one or more specific antibodies can determine the identity of the synthetic protein. For example, if one terminus of the protein contains a random 8 amino acid sequence tag and a library of SCAs has been constructed and enriched for SCAs binding to an equivalent mixture of synthetic peptides, then individual or small groups of SCAs will bind to specific peptides and the peptides (and therefore synthetic proteins) can be identified by knowledge of the specificity of individual SCAs.

Encompassed by the present invention are also methods where mixtures of modified proteins or interacting proteins are preselected prior to analysis of differences between different tissues or cells. For example, primary synthetic proteins created by display methods such as ribosome display where individual mRNA molecules encoding a protein remains attached to the protein are subjected to treatment by tissue or cell extracts and then subjected to selection using antibodies against these modifications immobilised on a solid phase. Thus, modified proteins with associated nucleic acids are isolated and the profile of proteins isolated can be determined by several means including a simple analysis of PCR products, for example by agarose gel electrophoresis, and comparison of results from treatments with different cells and tissues. Similarly, for primary natural proteins which are then used to "capture" secondary synthetic proteins by protein-protein binding, interacting proteins could be isolated using, for example, an antibody against a tag on the secondary synthetic protein and a profile of the interacting proteins determined again by analysis of PCR products from the nucleic acids associated with the synthetic protein.

Encompassed by the present invention are also methods where synthetic proteins generated from gene libraries are subjected to protein gel electophoresis for the analysis of protein modification or protein-protein binding. It is well documented that post translational modifications such as proteolysis or phosphorylation change the electrophoretic mobility of a protein (Phizicky & Fields Microbiol. Rev., 59, (1995), pp94). One embodiment of the invention therefore provides a method whereby disease associated protein modifications would be detected using 2D gel electrophoresis. For example, a cDNA library for subsequent IVTT or ribosome display would be constructed. To facilitate subsequent purification of the proteins, a 3 tag such as His or Flag may be incorporated into the transcription vector used. This will be achieved by standard molecular biology techniques that will be familiar to those skilled in the art. To facilitate subsequent detection of the proteins, they may be labelled during the translation process with labels such as 35S or biotin. Following translation, the proteins could if required be purified to remove ribosomes and other factors for example by passage of the translation mixture over an affinity matrix containing ligands such as anti-Flag antibodies or nickel where the proteins have 3 flag or ployhistidine tags or using anti-ribosome antibodies to remove ribosomal components onto a solid phase or by simple ultracentrifugation of ribosomes. Protein from a clinical sample (either unlabelled or labelled, for example through reaction with a fluorescent moiety) would then be incubated with the purified in vitro translated library to effect either modification or protein interactions withy the synthetic protein. The total reaction would then be separated by 2D gel electrophoresis (Cash, J. Chromatography 698 (1995), p203) and the resultant gels analysed using appropriate computer software such as Phoretix-2D (Phoretix International, Newcastle upon Tyne, UK). A control reaction would be performed such that the synthetic protein library would be reacted with total protein from an alternative clinical sample, for example a normal sample if the first sample is a diseased sample. Disease associated modifications or protein/protein interactions would be identified as unique bands seen with the diseased proteins and not seen in the control proteins. In order to facilitate rapid identification of the gene associated with protein identified by the 2D gel analysis, the synthetic protein library could initially be sub-divided into a number of pools, each pool containing a proportion of the library. This would be achieved by dividing the initial mixture of genes. Each pool would then be screened as described above to identify a pool containing a protein of interest. The transcription mix for that pool would then be sub-divided again and the screening process repeated. This procedure would be repeated using progressively smaller pools of ribosome displayed proteins until a single clone was identified which encoded the protein of interest. Alternatively, following identification of a potentialdisease associated protein by 2D gel analysis, the protein would be purified from the gel according to standard protocols (Hager & Burgess, Anal. Biochem., 109, (1980), pp76) and the purified protein panned with a scFv library to identify an antibody(s) which recognises the protein. The antibody(s) would then be used to screen a synthetic protein library constructed from the cDNA of the diseased sample to identify the protein with its associated gene tag. As an alternative highly innovative method to identify the protein, each recombinant protein may be produced with an amino acid sequence tag originally encoded by the plasmid vector and subsequently incorporated into the protein, for example by incorporating a leader sequence into the cloned cDNAs derived from the vector or from a mixture of synthetic oligonucleotides used to copy the cDNA for subsequent cloning into the vector. The nucleotide sequence tag may be variable or randomised in the plasmid vector preparation, for example by producing the plasmid vector using a synthetic region encoded by a random oligonucleotide mixture (with appropriate ends which anneal to the vector) to place random a sequence tag at, for example, the 5 end of the mRNA. Following production of the protein mixture by, for example, IVTT or ribosome display and separation of these proteins by 2D gel electrophoresis, individual proteins could then be analysed by probing with antibodies specific for the various permutations of sequences.

It will be obvious for the present invention that a prime application will be in the analysis of proteins, protein modifications and protein-protein interactions which relate to human disease or to the healthcare, individual or drug treatment status of humans. The invention incorporates the use of "clinical samples", such term referring to samples which may be tissue, blood or other which could be expected to be affected by the particular disease. The diseased samples refers to a sample which can be demonstrated to be affected by a disease, healthcare status or drug treatment status. The normal sample refers to a sample which is not affected by the disease and represents normal healthcare status, but may vary between individuals and may be relevant to the subsequent outcome of drug treatment. For comparative purposes in searching for disease associated proteins or protein modifications/interactions, the normal and diseased samples would ideally be matched to reduce population induced heterogeneity. This may be achieved by obtaining normal and diseased samples from a single patient (e.g. cancerous breast tissue and unaffected breast tissue) or alternatively by pooling diseased and normal samples from a number of different patients.

The generation of cDNA libraries from both normal and clinical samples is performed by standard techniques involving the isolation of mRNA followed by reverse transcription to generate cDNA (Sambrook et al., Molecular Cloning : a Laboratory manual, 2nd Ed., Cold Spring Harbor Press, 1989) or alternatively may be achieved using commercially available kits e.g. PolyATract System (Promega, Southampton, UK). The cloning of the cDNAs into a vector suitable for generation of synthetic proteins such as in vitro display by ribosome display and the conditions for in vitro display of the proteins would be as previously described (for example in Hanes and Pluckthun, Proc. Natl. Acad. Sci. 94 (1997), p4937). The isolation of total protein from the samples would be achieved by standard methods as detailed Bollag & Edelstein (Protein Methods, Wiley-Leiss, 1991). Ribosome Display - In the second embodiment of the present invention, there is provided a new in vitro method of ribosome display which maximises the expression of full-length polypeptides from a ribosome by selective arrest of mRNA translation at the 3' end of the mRNA such that the ribosome complex it is still linked to the full-length polypeptide. The invention is based on the discovery that proteins bound to specific sites in mRNA molecules will block the further translation of the mRNA allowing the stalling of a mRNA-ribosome complex with associated polypeptide. The invention also includes optional measures to prevent new translational starts just prior to polypeptide screening.

The method of the present invention involves firstly the creation of a library of DNA molecules, commonly within a plasmid vector, such DNA molecules encoding various polypeptides whereby the DNA encoding these polypeptides is transcribed into mRNA molecules. Commonly, the cloning vector for the DNA molecules includes an upstream promotor such as a promotor for T7 RNA polymerase. Thus, the pooled library of DNA molecules is transcribed, for example by the addition of T7 RNA polymerase and ribonucleotides, to produce a library of mRNA molecules. Thereafter, the library of RNA molecules is translated using a ribosome preparation such as an E.coli S-30 fraction (Chen H Z and Zubay G, Methods in Enzymology, vol 101 (1983) p674-690). Thereafter, the ribosome complexes, comprising linked polypeptide, ribosome and mRNA, are typically screened for binding to a ligand immobilised onto a solid phase and mRNA is released from the resultant immobilised complexes for reverse transcription and amplification by PCR in order to enrich for DNA molecules encoding polypeptides which bind to the target ligand. These DNA molecules can either be used directly for transcription or can be cloned in order to determine the sequences of DNA encoding polypeptides which bind to the target ligand. For the purpose of this embodiment of the invention, the mRNA encoded by the DNA molecules will be considered to include 3 segments comprising, from 5' to 3', a variable segment, a spacer segment and a termination segment with an optional anti-initiation segment 5' to the variable segment. The polypeptide shall mean the protein or peptide sequence translated from the variable segment of the mRNA to protein via a ribosome complex. The variable segment shall mean mRNA sequences encoding the full-length polypeptide. The spacer segment shall mean mRNA sequences encoding protein segments contiguous with the variable segments which allow the completed proteins to completely emerge from the ribosome and adopt optimal three dimensional structures whilst still attached to the encoding mRNA through the ribosome complex. The terminating segment shall mean mRNA sequences to which the binding moiety attaches either directly or indirectly or, alternatively, mRNA sequences encoding a polypeptide binding moiety, contiguous with or upstream from the spacer segment to which specific proteins attach in order to block translation. The binding moiety shall mean any molecule that can bind either to the mRNA on the ribosomal complex or any molecule that can bind to the translated polypeptide on the ribosomal complex resulting in arrest of translation. The binding moiety will usually bind directly to mRNA in a sequence specific manner or may be bound indirectly to the mRNA, for example after annealing of a synthetic DNA or RNA molecule to the mRNA and addition of the binding moiety via a ligand on these molecules. The optional anti-initiation segment will be adjacent to the translational initiation codon and will provide sequences for attachment usually of a different binding moiety. The anti-initiation segment will prevent new translational initiations just prior to screening of the translated polypeptides. The target ligand will be the molecule against which the library is screened for binding of specific proteins and subsequent recovery of associated mRNA.

The variable segment of the mRNA molecules comprises either full-length known polypeptide types such as single-chain antibodies (SCAs, comprising immunoglobulin variable regions derived from heavy and light chains and linked together to give a functional binding domain), or random or semi-random sequences. Chimaeric sequences created by random/semi-random association of known polypeptide types or regions may also be used. For known polypeptide types such as SCAs, specific segments of the genes may be randomised such as the CDRs in SCAs. Large collections of DNA molecules encoding known, random or semi-random sequences will be firstly produced and then cloned into the transcription vector. This transcription vector will provide other segments for inclusion in the subsequent mRNA molecules as detailed below. The vector will also usually provide a translation initiation codon and ribosome binding site for the mRNA. For longer polypeptide molecules encoded by the DNA, it will be necessary to reduce or eliminate the presence of stop codons in the mRNA which would terminate translation. It is a requirement of the invention herein that such stop codons, including stop codons natural to specific proteins, are eliminated in order to yield full-length polypeptides.

For DNA encoding known proteins such as single-chain antibodies, this will simply require elimination of the usual stop codon or, alternatively, the use of nonsense suppressing tRNAs in the translation reaction in order to insert specific amino acids at the position of the stop codons. For mixtures of random or semi-random DNA molecules produced by chemical synthesis, the frequency of stop codons occurring in any particular sequence can be reduced by manipulation of DNA base composition in the synthesis reaction and nonsense suppressing tRNAs could also be used in the translation reaction. The spacer segment of the mRNA molecules provides a polypeptide region downstream of the variable segment which spans the channel of the ribosome in order to permit the full-length polypeptide to fully emerge from the ribosome to allow for proper protein folding and unhindered access to the target ligand. Usually this segment will encode a region of over 50 amino acids and will encode a region which is unlikely to interfere with folding of the full-length polypeptide such as a complete domain from another protein or a glycine/alanine-rich linker such as (Gly4Ser)10. For some translated proteins, the spacer segment may comprise a contiguous portion from the protein itself where this portion is not required for correct folding of the variable segment or for binding to the target ligand.

The terminating segment provides a downstream mRNA sequence for direct or indirect attachment of a binding moiety which is designed to prevent translational termination following translation of the complete variable segment. A particularly favoured method for attachment of a binding mioety is indirectly using an intermediate synthetic oligonucleotide molecule which first anneals to the terminating segment. If such synthetic nucleotide is equipped with a specific binding site for the binding moiety, then the binding moiety can, in turn, bind to the annealed synthetic oligonucleotide and thereby attach to the downstream end of the mRNA to block translation. One example of this favoured method is to use an oligonucleotide which incorporates one or more biotinylated nucleotides such that streptavidin can subsequently bind thus providing a molecule which blocks translation. The use of molecules such as streptavidin also provides for simple possibilities for cross-linking the nascent protein to the mRNA molecule where the nascent protein could itself be biotinylated by virtue of incorporated biotinyl lysine (or another biotinylated amino acid) in the in vitro translation reaction mixture. Another example of this favoured method is to use an oligonucleotide which incorporates one or more nucleotides derivatised with a ligand such as fluorescein or dinitrophenol which can then be bound by a monoclonal antibody or fragment thereof. This also makes feasible the use of bispecific molecules such as bispecific antibodies which might bind, on the one hand, to the mRNA via the synthetic oligonuleotide, and on the other hand also to the translated protein or polypeptide by virtue of an amino acid sequence on the translated protein or poypeptide. Other methods include providing the terminating segment with a sequence to which a specific protein binds directly without any intermediate annealing step. One example of such a binding moiety is the Iron Regulatory Protein (IRP). The terminating segment would provide a stem-loop structure which is stabilised by the addition of the IRP in conditions of low iron concentration, thus resulting in a steric block to the ribosome translation. Following this, selection of the nascent peptide can occur. The use of IRP to arrest the ribosome introduces a reversible block such that addition of iron may allow translation to resume and thus terminate. This would facilitate the release of the mRNA after selection of the polypeptide for subsequent transcription, sequencing or cDNA cloning. Further examples of mRNA binding moieties binding to sequences in the terminating segment include the HIV protein tat, which binds to a RNA stem-loop termed TAR (Dingwall et al., PNAS, vol 86 (1989) p6925-6929), La antigen which binds to a RNP motif (Chan E K L and Tan E M, Mol. Cell. Biol. vol 7 (1987) p2588-2591) and other proteins from RNA viruses which bind to specific RNA sequences either in single- or double- stranded RNA, the latter which can be created in mRNA via hairpin loops.
The terminating segment alternatively could encode a site for attachment of a binding moiety which additionally binds to the synthesised polypeptide itself or to the ribosomal protein complex in order to further stabilise the binding moiety for prevention of translational termination. Optionally, the terminating segment will include one or more further regions of mRNA which increase the stability of mRNA against exonucleases such as the lpp (E.coli lipoprotein) and phage T3 terminators. It will be particularly clear to those skilled in the art that, for the major aspect of this embodiment of the invention, mRNAs could be produced with a variety of terminating segments to which a variety of binding moieties could bind either indirectly, through the initial annealing of a synthetic oligonucleotide or other molecule, or directly through the recognition and binding of a binding moiety directly to the mRNA molecule.

The optional anti-initiation segment adjacent is designed to prevent translational initiations late in the translation reaction which might not result in full-length polypeptides and might therefore provide polypeptides with incomplete folding which might provide non-specific binding to target ligands. The anti-initiation segment might encode, for example, a secretory leader sequence in the translated polypeptide to which can be bound the signal recognition protein (SRP). Once SRP has bound to the polypeptide, it causes the arrest of further translation by cross-linking of the polypeptide and the mRNA. Translational arrest may also be achieved by using a combination of the SRP54 and SRP9/14 subunits of SRP (Siegel & Walter, Cell Biol., vol 100 (1985) 1913-1921) which bind to the nascent peptide and mRNA respectively. Another example of anti-initiation sequences is provided by certain eukaryotic transcription leaders such as that of the human cytomegalovirus gp48 gene which contains an upstream 22 codon which represses translation of the downstream cistron (Cao J and Geballe A P, Mol. Cell. Biol. vol 16 (1996) p7109-7114). Such leaders are thought to encode peptides which block ribosome progression to the downstream cistron. Further examples of anti-initiation sequences are sequences recognised by binding moieties such as for the terminating segment as above, these including sequences bound by IRP, tat, La antigen and other viral proteins.

The invention therefore provides compositions of DNA libraries encoding mRNA molecules with variable, spacer and terminating segments with an optional anti-initiation segment. Preferably, the invention provides a DNA vector encoding the spacer and terminating segments; the anti-initiation segment is optional. Commonly, the DNA vector will also provide the translation initiation codon and, for translations using prokaryotic ribosomes, a ribosome binding site including the Shine-Dalgarno sequence. For eukaryotic translation systems, the Kozak translation initiation sequence with consensus GCCGCCACCATGG may also be included and, upstream from the translation initiation site, it may be desirable to include other known sequences to enhance translation such as enhancers or activator sequences including untranslated leader sequences from certain viruses such as tobacco mosaic virus. Commonly, the DNA vector will also provide a strong transcriptional promotor which will be used in conjunction with a RNA polymerase to produce a library of mRNA molecules corresponding to the DNA library. Such promotors will include those for T7 RNA polymerase, T3 RNA polymerase and SP6 RNA polymerase and, additionally, a promotor for the RNA dependant polymerase, Qb replicase, may also be encoded by the DNA. The DNA vector will also provide a strong transcriptional terminator, for example the terminator ofE coli lipoprotein or the early terminator of phage T3. In the method of the invention, DNA fragments containing the variable segments are cloned into the DNA vector whereby the DNA fragments have a minimum of or no stop codons. For libraries encoding known polypeptide types such as SCAs, the DNA fragments will be cloned unidirectionally using appropriate restriction sites. It will be apparent to those skilled in the art that a variety of replicable DNA vectors could be used in the method of the present invention including plasmid, bacteriophage, phagemid and viral vectors. It will also be clear that DNA amplification by methods such as PCR could be used as an alternative to replication of DNA in living cells. It will also be clear that the variable segments to create the DNA library could be provided from a number of sources including a vector library of DNA fragments, synthetic DNA or amplified DNA. In addition, the variable segments could be provided as a result of mutagenesis reactions using a fixed template, for example using error-prone PCR. It will also be clear that the variable segmeIRPnts could be composed of DNA directly from the genome of a living organism or from cDNA copies of mRNAs of that organism. For example, where the DNA library comprises single-chain antibody fragments, these fragments could be derived from mRNA encoding immunoglobulin variable regions and expressed by B cells within an organism. Alternatively, the fragments could be derived from genomic variable regions. In such manner, the invention will provide for the creation of single-chain antibody libraries from specific organisms such as man.

The invention also provides compositions of libraries of mRNA molecules with variable, spacer and terminating segments with an optional anti-initiation segment. Preferably, the invention provides a library of mRNA molecules encoding the spacer and terminating segments, with the anti-initiation sequence optional. The mRNA molecules will each include a translation initiation codon and, for translations using prokaryotic ribosomes, a ribosome binding site including the Shine-Dalgarno sequence. For translation using eukaryotic ribosomes, the mRNA may synthesised with a 5' capping nucleotide or this may be introduced enzymatically into the pre-synthesised mRNA Upstream from the translation initiation site, the consensus Kozak translation initiation sequence may also be included and other known sequences to enhance translation such as enhancers or activator sequences including untranslated leader sequences from certain viruses. Prior to or during the process of translation, one or more binding moieties will become associated with the terminating segment of the mRNA molecules thus stalling translation. Also, during the process of translation, one or more binding moieties may become associated with the optional anti-initiation segment within or encoded by the mRNA molecules thus preventing new translational initiations.

The invention also provides compositions of libraries of translated polypeptide molecules with variable and spacer segments with an optional anti-initiation leader sequence. These protein molecules will provide full-length variable segments as part of a longer polypeptide chain which is stalled within the ribosomes through the interaction of binding moieties with the mRNA.

The present invention relates toacts with the ribosome thus preventing additional translation on the mRNA molecule. Alternatively, the 5' end may become associated with a binding moiety indirectly such as via the annealing of a synthetic oligonucleotide to a complementary sequence at the 5' end and subsequent binding of a binding moiety to this oligonucleotide, such as via biotin molecules incorporated into the synthetic oligonucleotide.

Within the method of the present invention, it will be apparent to those skilled in the art that various measures could be used to optimise the stability of mRNA molecules especially from degradation by RNAses. For example, various inhibitors ofRNAses such as Rnasin and vanadyl ribonucleoside complexes could be used in the transcription reactions.

Alternatively or additionally, various structures could be included in the mRNA molecules including 3' stem-loops such as those commonly provided by transcriptional terminators. It will also be apparent to those skilled in the art that transcription and translation reactions might either be performed separately or, especially with prokaryotic systems, combined into a coupled in vitro transcription/translation reaction. Preferably, the transcription and translation reactions will be performed separately in order to optimise the production of mRNA and polypeptides which will require different optimal reagents.

Within the method of the present invention, it will be apparent to those skilled in the art that various measures could be used to optimise the folding and stability of protein molecules especially from degradation by proteases. For correct protein folding, optimal oxidative protein folding conditions would be used in translation reactions with particular measures to optimise disulphide bond formation through, for example, use of molecular chaperones such as protein disulphide isomerase. For stability, the peptide tagging system of E.coli could be disabled by inhibition of ssrA RNA using methods such as that of Hanes and Pluckthun (ibid).

Protein-Protein Interactions- This embodiment of the present invention provides methods for isolating genes encoding displayed proteins or polypeptides which bind to a ligand by providing molecular tags on both protein/polypeptide and ligand as the basis for isolating the gene. The method particularly includes isolation of genes where the ligand is itself a displayed protein/polypeptide. This embodiment of the invention is based upon the binding of a polypeptide to a ligand (or other polypeptide) whereby molecular tags on the polypeptide and ligand can be used as the basis for molecular separation of the bound polypeptide-ligand from unbound polypeptide and ligand whereby the polypeptide is still associated with it's gene (or gene transcript). For selection of a polypeptide which binds to a ligand, a particularly favourable method is to provide molecular tags on both the polypeptide and it's ligand whereby methods are then applied for isolation of complexes containing both molecular tags. For example, using the technique of ribosome display, the mRNA may be tagged with a RNA binding protein (such as HIV tat protein) whilst the ligand (if a polypeptide) may be tagged with a polyhistidine tail such that consecutive passage of a library of mRNA-ribosome-polypeptide complexes over affinity matrices comprising anti-tag antibodies and nickel would select for binding polypeptides with associated mRNA which can then provide the gene sequence encoding for binding polypeptides. Alternatively, using ribosome display, only the polypeptide may be tagged, for example with a polyhistidine tail, passed over affinity matrices such as nickel chelate and any associated RNA may be accessed simply by converting into cDNA copies by PCR. For selection of 2 or more binding polypeptides, a particularly favourable method is to use the technique of ribosome display using di(or poly)cistronic mRNAs for generation of candidate binding polypeptides derived from one or more DNA libraries. If the cistron for one of the polypeptides is constructed such that the translated polypeptide is dissociated from mRNA after translation and the cistron for the other polypeptide is constructed such that the translated polypeptide remains associated with mRNA after translation, then the translational juxtaposition of the 2 polypeptides is such that association, if applicable, is facilitated by this juxtaposition thus providing a molecular basis for separation of the complexes including the binding polypeptide(s) and mRNA, the latter which can then be used for the identity of genes encoding the binding polypeptide(s).

Another aspect of this embodiment of the present invention is based upon the binding of 2 polypeptides, one or both of which are associated with the corresponding gene, which are fused (or associated) with other polypeptide chains which, when juxtaposed, will generate a selectable property which then allows isolation of live microorganisms encoding the gene(s) for the binding polypeptides(s). Of particular interest within this embodiment is the generation of enzyme activities by the association of 2 polypeptides. Such generated enzyme activities can be used in a variety of ways for selection. These include enzyme activities which generate molecular tags which attach to complexes of successfully paired polypeptides and associated genes (or mRNA). For polypeptide display on microorganisms, these also include enzyme activities which generate (or release) molecules in the vicinity of the microorganisms which leads to the selection of microorganisms displaying a polypeptide derived from a DNA library which binds to a ligand. Also of interest within this embodiment is the generation of binding protein activities by the association of 2 polypeptides whereby the binding protein indirectly generates a selectable property which can be used as a basis for identifying the gene encoding the binding protein(s). For example, the binding protein could be a DNA binding protein such as a transcriptional activator which activates a gene which provides a selection advantage to the microorganism such as a antibiotic resistance protein or a nutrient generating protein.

For application of the present invention to binding polypeptides displayed on microorganisms, the invention provides for methods where the ligand (or second polypeptide) is added externally to the microorganism or methods where the ligand is synthesised internally within the microorganism, in both cases where the association of the polypeptide and ligand can generate a new molecule (or molecules) which provides a basis for selection or separation of the microorganism displaying the binding polypeptide. The invention can be based upon the complementation of an enzyme activity through the combination of 2 (or more) subunits of an enzyme which, when associated together, reform the enzyme activity. Reformation of the enzyme activity will then either provide for the positive or negative selection of the microorganism providing the enzyme activity. In one example of this embodiment of the invention, there is provided a DNA library encoding variant polypeptides which can be expressed in a microorganism or a cell whereby the genes encoding the binding polypeptides are fused to a portion of the gene encoding an enzymatically inactive fragment of an enzyme whereby this portion of enzyme can complement another portion of the enzyme to reconstitute enzyme activity. Suitable enzymes include E. coli beta-galactosidase and C. perfringens phospholipase C. The other portion of the enzyme is either provided in the microorganism culture medium for selection in vitro or provided by expression of a gene encoding this portion within the same microorganism for selection in vivo. Enzymes such as E. coli beta-galactosidase and C. perfringens phospholipase C have the property that inactive subunits of the enzyme can self-associate in solution to reconstitute enzyme activity and thus the invention provides for a situation whereby the binding polypeptide, fused to the gene encoding an inactive enzyme fragment, can bind to its ligand and either promote (where the ligand is fused to the other inactive subunit) or prevent (where the polypeptide-ligand binding, through steric hindrance, prevents the reconstitution of enzyme activity by the inactive enzyme subunit. The restoration or abolition of enzyme activity may have a beneficial or deleterious effect on the microorganism expressing the polypeptide / enzyme subunit gene fusion. This will either be due to the direct enzymatic action of the reconstituted enzyme, for example in causing the conversion of a non-toxic substrate to a toxic product, or indirect enzymatic action of the reconstituted enzyme, for example in causing the lysis of external liposomes containing a toxic agent or an essential nutrient/factor for growth/propagation of the microorganism.

The following examples are provided to illustrate the invention and should not be considered as limiting the scope of the invention.

### Example 1

### Generation of Synthetic Protein Libraries and Modification by Tissue

The starting point was a cDNA library preparation from "marathon-ready" human colon cDNAs obtained from Clontech UK Ltd (Basingstoke, UK). The library was amplified by PCR using primers AP1 [5'ccatcctaatacgactcactatagggc] and AP3 [5'ttctagaattcagcggccgc(t)₃₀nn] using the Advantage cDNA PCR kit and conditions recommended by the supplier (Clontech). The resultant PCR product was cloned using a T/A cloning strategy into SmaI linearised pUC19 prepared according to Marchuck et al (Marchuck D. et al 1991, Nucl.Acids Res. **19**: 1154). Alternatively, a human colon cDNA library constructed from human colon mRNA isolated directly from tissue using standard methods described in *Molecular Cloning, A Laboratory Manual* eds. Sambrook J, Fritsch EF, Maniatis T. Cold Spring Harbor Laboratory Press 1989, New York, USA. The mRNA was converted to cDNAs using the RNAse H method (described in *Molecular Cloning* ibid) and recloned into the SmaI site of pGEMT7/SP6 plasmid (Promega, Southampton, UK) providing a promoter for T7 RNA polymerase. Long synthetic oligonucleotides and PCR were applied to provide an upstream bacterial ribosome binding site, a downstream spacer derived from the M13 phage gene III and a 3' transcriptional terminator region from the *E. coli* lpp terminator as described in Hanes and Pluckthun, *Proc. Natl. Acad Sci.* 94 (1997), p4937.

*In vitro* transcription of the pT7 plasmids was performed using a RiboMAX™ kit (Promega, Southampton, UK) according to the manufacturer's instructions. The resultant mRNA was purified according to the manufacturer's protocol. *In vitro* translation was performed using *E coli* S30 Extract System with the inclusion of ³⁵S methionine (Promega, Southampton, UK) according to the manufacturer's instructions. The ribosomes were dissociated from the proteins by treatment with EDTA as described by Hanes & Pluckthun (ibid) and the ribosomes removed by centrifugation (100,000 g for 30 min).

Crude protein extracts were prepared from a normal colon and a colorectal carcinoma sample by disruption of the tissue in ice cold RIPA buffer (RIPA buffer = 50mM Tris-HCL, pH7.4, 1%(v/v) NP-40, 0.25% sodium deoxycholate, 150mM NaCl, 1mM EGTA, 1mM PMSF, 1mM Na₃VO₄, 1mM NaF and 1ug/ml each of aprotinin, leupeptin and peptstatin). In some experiments, extracts competent for phosphatase activity were also produced; in this case lysates were produced using RIPA buffer without 1mM Na₃VO₄. Tissue samples were thawed on ice and disrupted using a sterile disposable tissue grinder ("pellet pestle" Anachem Ltd, Luton, UK). Homogenisation was conducted using 2 volumes of ice cold RIPA buffer to 1 volume of tissue. The lysate was incubated with gentle shaking on ice for 15 minutes then cleared of insoluble material by centrifugation at 13,000 x g at 4°C for 10 minutes. The supernatant was removed and assayed for protein concentration using the bicinhoninic acid method and protocols provided by the supplier (Pierce, Chester UK, cat#23225). The lysate was divided into multiple aliquots, snap-frozen on dry ice and stored in liquid nitrogen.

Normal colon and colorectal tumour lysates were reacted with the *in vitro* translated proteins by incubation of thawed lysate for 30 minutes at 37°C in the presence of 1 mg/ml vanadyl ribonucleoside complexes and 10 units Rnasin. The protein mixture was then solubilised according to the protocol of Cash *et al*., (*Electrophoresis* 1**8** (1997), p2580). The soluble proteins were then analysed by 2D PAGE (Cash *et al.* ibid) and the proteins detected by autoradiography (Patton *et al.*, *BioTechniques* **8** (1990), p518). Images of the gels were captured using a video camera connected to an image analysis system and then transferred to Phoretix-2D for further analysis. Analysis was performed using Phoretix-2D systems according to the manufacturer's instructions.

Out of a total of 2622 protein spots detected from the *in vitro* translated protein mixture, 12 spots demonstrated migration differences when treatments with normal and colorectal cancer extracts were compared.

### Example 2

### Phosphorylation of Synthetic Proteins

Recombinant purified rat ERK-2 containing a polyhistidine tag fused to the amino terminus of the protein was purchased from Stratagene, La Jolla, USA (cat # 20612). This product is the non-activated form of the protein and contains no phosphorylated residues. 0.5ug non-activated ERK-2 was reacted with a 5µl (containing 2mg/ml total protein) of A431 cell lysate (Upstate Biotechnologies, Lake Placid, NY, USA cat #12-110) or alternatively produced in-house from cells grown in tissue culture. The procedure of reacting a cellular or tissue lysate with a target protein is referred to as the Conditioning Reaction.

Cell lysates were produced by suspending approximately 5x10⁶ sub-confluent cells in 500µl of ice cold RIPA buffer. Cells had been previously washed of medium using ice cold PBS and were incubated with gentle shaking on ice for 15minutes in RIPA buffer. The lysate was cleared of insoluble material by centrifugation at 13,000 x g at 4°C for 10 minutes. The supernatant was removed and assayed for protein concentration using the bicinhoninic acid method and protocols provided by the supplier (Pierce, Chester UK, cat#23225). The lysate was divided into multiple aliquots, snap-frozen on dry ice and stored in liquid nitrogen before use in the conditioning reaction. Lysates from tissue samples and other cell types were processed in the same way.

The conditioning reaction was carried out at 37°C for 15 minutes and then placed on ice. The reaction was divided equally (by volume) into two tubes for detection of phosphorylated ERK-2 using either magnetic sorting (tube 1) or binding to microtitre plate wells (tube 2) in both cases using anti-histidine antibodies as capture reagent.

In tube 1, ERK-2 was captured using magnetic beads (Dynal, Wirral UK, cat#110.11) pre-coated with an anti-histidine antibody (Sigma, Poole UK, cat#H1029) using coating conditions recommended by the supplier. The capture reaction was carried out at 4°C 1 hour with constant gentle mixing throughout to maintain the beads in suspension. Following capture, the beads were collected using a magnetic particle concentrator and washed extensively in PBS. Beads were resuspended in a final volume of 40µl and 10µl aliquots were taken into microtitre plate wells and individually reacted with dilutions of either anti-phosphorylated ERK antibody (Upstate Biotechnologies, cat#05-481), anti-phosphotyrosine-HRP conjugate monoclonal cocktail (Zymed, Cambridge UK, #136620), anti-phosphoserine /phosphothreoinine /phosphotyrosine polyconal preparation (Zymed, cat#90-0200) or a negative control antibody-HRP conjugate specific for human immunoglobulin light chains (The Binding Site, Birmingham UK, cat #APO015). Antibodies were incubated for 1 hour at 4°C before detection with respective secondary reagents /chromogenic substrates. Plates were read at 450nm.

In tube 2, ERK-2 was captured using microtitre plate wells which had been coated overnight with an anti-histidine antibody (Sigma, Poole UK, cat#H1029), and pre-blocked by 40 minute incubation with a solution PBS/5%(w/v) BSA at room temperature. The conditioning reaction was diluted to a total volume of 100ul using PBS and added to the microtitre plate. The capture reaction was for 1 hour at room temperature. Phosphorylated ERK-2 was detected in direct ELISA format using the panel of antibodies including a negative control reagent as given above.

Using both detection methods above, the presence of phosphorylated ERK-2 was unequivocally demonstrated following incubation with lysates from A431 cells.

### Example 3

### Method for preparing in vitro translated cDNA clones for arraying onto streptavidin coated plates.

A human whole foetal brain cDNA library was obtained from Stratagene Cloning Systems (La Jolla, USA). The library was supplied cloned unidirectionally into the lambda vector Uni-ZAP XR with an average insert size of 1.3kbp. The library was estimated to contain 2x10⁶ pfu and was supplied at a titre of approximately 2.4x10¹⁰ pfu/ml. The master lambda Uni-ZAP XR library was supplied following a single round of amplification. A mass excision reaction (below) was conducted on this 1x amplified material to enable manipulations such as plating, replicate production and banking of master stocks to be conducted at the level of individual clones of viable E.*coli* and not as lytic plaques following phage lambda infection (Jerseth, B. et al (1992) *Strategies* 5: 81-83). The remaining library was further amplified once using standard procedures (*Molecular Cloning, A Laboratory Manual* eds. Sambrook J, Fritsch EF, Maniatis T. Cold Spring Harbor Laboratory Press 1989, New York, USA) and multiple glycerol stocks laid down as a master stock bank at -80°C.

The library was prepared for a mass excision reaction and plating as bacterial colonies according to protocols provided by the supplier (Stratagene, La Jolla, USA). Briefly, bacterial strains (XL-1 Blue and SOLR') supplied with the library were plated on selective medium (LB tetracycline and LB kanamycin respectively) and single colonies picked for overnight culture at 30°C in 50ml of LB broth [10g/l NaCl, 10g/l tryptone, 5g/l yeast extract, pH 7.0] supplemented with 0.2% (w/v) maltose and 10mM MgSO₄. Cells were collected by centrifugation and re-suspended in 10mM MgSO₄ at a density of 8x10⁸ cells/ml (OD₆₀₀ = 1.0). XL-1 Blue cells were combined with a portion of the lambda Uni-ZAP XR library at a multiplicity of infection of 1:10 lambda phage:cells. M13 based helper phage strain ExAssist (Stratagene, La Jolla, USA) was added at a ratio of 10:1 helper phage:cells and the mixture incubated at 37°C for 15 minutes. 20ml of LB broth was added and the mixture incubated at 37°C with shaking for a further three hours. The mixture was heated at 70°C for 20 minutes and the cell debris collected by centrifugation. The supernatant containing the excised phagemids was collected and 1µl was added to 200µl of SOLR' cells. The mixture was plated onto LB ampicillin plates and incubated overnight at 37°C. Typically many 1000's colonies resulted on each plate and further dilution of the excised phagemid preparation was required to get suitable plating densities for colony picking.

In order to make feasible to gridding process, the total number of individual cDNA clones was reduced whilst maintaining representation of the library. This normalisation was achieved following the procedure of Soares et al (Soares, M. B. et al 1994, Proc. Natl. Acad. Sci USA vol 91: 922-923). The procedure required the production of single-strand molecules by helper-phage infection of the phagemid library. The single strand circles were subjected to controlled primer extension followed by denaturing and reannealing under conditions whereby high abundance molecules re-associated (low Cₒ*t*) and could be removed from the mixture by hydroxyapatite column chromatography. Non-reassociated circles were recovered from the flow through and transformed directly into competent bacteria.

Single-strand rescue was conducted on the phagemid library using helper phage VCM13 and protocols provided by the supplier (Stratagene). Rescued single-stranded DNA was purified according to the method of Soares et al (Soares et al ibid) incorporating particular steps to eliminate contaminating double-stranded DNA. Controlled primer extension was conducted on purified single-stranded circular DNA using primer 5'ggaaacagctatgaccatg, using the conditions of Soares et al. DNA polymerase was from Boehringer, nucleotide triphosphates were from Life Technologies (Paisley, UK). a ³²P dCTP used as tracer was from Amersham International (Amersham, UK). Hydroxyapatite column chromatography was conducted at 60°C as described in Soares et al. Following recovery of the normalised single-stranded circles, the purified DNA was directly transformed into competent XL1-blue E.*coli* cells and plated on LB-ampicillin plates as previously described.

Individual colonies of XL1-blue cells from the normalised library were picked for further analysis and replicate production. Briefly, colonies were manually picked into 96 well plates containing 100µl LB ampicillin medium and grown for 3 hours at 37°C. One replicate was made of each plate using a BioRobot9600 (Qiagen UK Ltd, Crawley UK) laboratory workstation. The robot was programmed to pipette 50µl of the culture into medium containing 10% (v/v) glycerol and this plate was sealed and stored as a master stock at -80°C. The remaining 50µl culture was inoculated into 96-well flat-bottom block with 2ml square wells containing 1.8ml LB-ampicillin medium. A microporous tape sheet was applied to the top of each block and the block incubated overnight at 37°C with orbital shaking. Following incubation, phagemid DNA was prepared from each of the 96 wells using standard programmes on a BioRobot9600 (Qiagen UK Ltd, Crawley UK) and the QIAwell Ultra reagent systems provided by the supplier (Qiagen UK Ltd Crawley, UK).

A portion of the purified DNA was used directly as templates for linked *in vitro* transcription translation (IVTT) using T3 RNA polymerase and a rabbit reticulocyte lysate system supplied by Promega UK Ltd (Southampton, UK). The remaining DNA was sealed for storage at -20°C. The IVTT reaction mix was supplemented with 2% (v/v) tRNA-biotinyl-lysine (Promega UK Ltd, Southampton, UK) to enable capture of *in vitro* translated proteins onto a streptavidin coated solid phase. IVTT reactions were conducted in 96 well arrays in a total volume of 25µl at 30°C for 60 minutes. Following incubation, 5µl each reaction was taken into 100µl TSB pH 8.0 (50mM Tris, 138mM NaCl, 2.7mM KCl) previously added to the wells of a Reacti-Bind™ steptavidin coated pre-blocked plate (Pierce, Chester, UK). The remaining IVTT reaction plate was sealed for storage at -20°C. The Reacti-Bind™ steptavidin plate was incubated at room temperature for 60 minutes with gentle rocking to promote binding of the biotinylated IVTT product to the plate surface. The plate was washed using 3x 200µl wash buffer (TBS, 0.1% (w/v)BSA, 0.05% (v/v)Tween 20) before use in tissue protein binding assays.

### Example 4

### Method for performing tissue extract modification of in vitro translated protein arrays and detection of phosphorylation of arrayed proteins.

Human brain tissue samples were obtained from NeuroResouce (London, UK). Matched samples of normal and Alzheimers disease brain were provided. In general 1-10g each sample was provided, in all cases samples were from known sub-anatomical location and cross-indexed to full clinical details. All samples were snap frozen at point of sampling and shipped on dry-ice and stored under liquid nitrogen before processing. Processing was conducted under Category 2 containment.

Phosphorylation competent brain tissue extracts were produced by disruption of the tissue in ice cold RIPA buffer. In some experiments, extracts competent for phosphatase activity were also produced; in this case lysates were produced using RIPA buffer without 1mM Na₃VO₄. Tissue samples were thawed on ice and disrupted using a sterile disposable tissue grinder ("pellet pestle" Anachem Ltd, Luton, UK). Homogenisation was conducted using 2 volumes of ice cold RIPA buffer to 1 volume of tissue. The lysate was incubated with gentle shaking on ice for 15 minutes then cleared of insoluble material by centrifugation at 13,000 x g at 4°C for 10 minutes. The supernatant was removed and assayed for protein concentration using the bicinhoninic acid method and protocols provided by the supplier (Pierce, Chester UK, cat#23225). The lysate was divided into multiple aliquots, snap-frozen on dry ice and stored in liquid nitrogen.

Brain tissue lysate was reacted with the IVTT protein array in 96-well format by incubation of thawed lysate with the washed array for 30 minutes at 37°C. In general 10µl lysate was added to each well of the protein array. Following incubation, the plates were washed x3 using 200µl wash buffer per well and incubated with any of several diluted anti-phosphoprotein antibody preparations. Phosphotyrosine modification was detected using anti-phosphotyrosine-HRP conjugate monoclonal cocktail (Zymed, Cambridge UK, #136620). Phosphoserine/phosphothreonine modification was detected using anti-phosphoserine /phosphothreoinine /phosphotyrosine polyconal preparation (Zymed, Cambridge UK, cat#90-0200). In all cases, antibody incubation was conducted using antibodies diluted in TBS containing 5%BSA, for a minimum of 1 hour at 37°C or in some cases overnight incubation at 4°C. Plates were washed as previously before detection with respective secondary reagents/chromogenic substrates following standard protocols (*Antibodies A Laboratory Manual* eds. Harlow, E. and Lane, D. Cold Spring Harbor Laboratory Press 1988 New York, USA). Plates were read at 492nm.

### Example 5

### Method for detecting protein-protein binding using labelled tissue proteins on in vitro translated protein arrays

The proteins of a brain tissue lysate were labelled with fluorescein using the FluroTag™ FITC conjugation system supplied by Sigma (Poole, UK). Typically 5mg brain protein was reacted with 250µl of a dilution of a 1mg/ml fluoroscein-isothiocyanate (FITC) solution in 0.1M carbonate-bicarbonate buffer. The labelling reaction was allowed to proceed for 2 hours at room temperature before purification of unincorporated FITC using G-25 Sephadex column chromatography. Columns and procedures were as provided in the FluroTag™ kit (Sigma, Poole, UK). The column eluate containing the labelled brain proteins was stored at 4°C protected from light.

Labelled tissue lysate was reacted with the IVTT protein array as previously. To detect labelled brain proteins captured by members of the IVTT protein array, the plate was washed as previously and incubated with an anti-fluorescein antibody (Sigma #F-5636, Sigma, Poole, UK). The antibody was diluted 1/2500 in TBS containing 5% BSA and incubated for 30-60 minutes at room temperature. The antibody was detected using an anti-mouse-HRP complex followed by reaction with the chromogenic substrate o-phenylenediamine (Sigma, Poole UK) according to standard procedures (*Antibodies, A Laboratory Manual,* ibid). Plates were read at 492nm.

### Example 6

### Protein-protein binding partners identified by ribosome display library

In this example, the *in vitro* translated protein array is used to search a ribosome display library for binding partners, and the genes for binding partners present in the library are identified by reverse-transcription polymerase chain reaction (RT-PCR). For the purpose of this example, the ribosome display library is essentially as described in example 1 and, in practice, at point of use for screening using the IVTT protein array, consists of a large population (10⁹-10¹²) of ribosomes each individually tethered to a nascent protein by linkage to its cognate mRNA molecule.

The library was diluted 1 in 5 in ice-cold dilution buffer (50mM Tris Acetate pH 7.5, 150 mM Sodium Chloride, 50 mM Magnesium Acetate, 0.1% Tween 20 with 200u/ml of RNasin (Promega UK Ltd, Southampton UK). 50µl of the diluted library was added to each well of the IVTT protein array and incubated at 4°C for 1 hour with gentle shaking. Following incubation, wells of the array were washed 5 times using 100µl ice-cold dilution buffer. The mRNA from any bound mRNA/ribosome/protein complexes was eluted by addition of 20µl of 50 mM Tris acetate pH 7.5, 150 mM Sodium Chloride, 20 mM EDTA and incubating on ice for 10 minutes with gentle shaking. Eluted mRNA was collected into a second 96 well plate and concentrated by precipitation with ethanol at -20°C overnight (Molecular Cloning, A Laboratory Manual ibid) in the presence 20µg of glycogen carrier, (Boehringer, Lewes, UK). The resulting mRNA was used as a template for reverse transcription with M-MLV reverse transcriptase (RT). Briefly, mRNA was heat denatured at 70°C for 10 minutes in the presence of 1nM of the primer RD3 [5'(T)₁₈nn]. The mixture was chilled on ice before addition of 200u M-MLV-RT and its reaction buffer (Life Technologies, Paisley, UK). The mixture was incubated for 60 minutes at 37°C then shifted to 70°C for 15 minutes to inactivate the RT. The subsequent DNA:RNA hybrid was used as a template for PCR, using primers T75L [5'cggtttccctctagaaata] and RD3 and cycled x 40 at 95°C for 30 seconds, 50°C for 30 seconds, 72°C for 30 seconds. Thermostable DNA polymerase (Expand™ High Fidelity), reaction buffer and nucleotide triphosphates were from Boehringer (Boehringer, Lewes, UK). PCR products were analysed by agarose gel electrophoresis (Molecular Cloning, A Laboratory Manual, ibid). The presence of a PCR product identified putative positive binding partners in the IVTT protein array. The protein array was de-coded and phagemid DNA identified for sequence analysis. PCR products from the polysome display library were sequenced directly using primer T75L. Sequencing was conducted using dye-terminator chemistry according to protocols provided by the supplier (Amersham International, Amersham UK). Reactions analysed using an automated sequencing system (Applied Biosystems, Warrington, UK).

### Example 7

### Microarrays of in vitro translated protein library onto a streptavidin coated glass surface.

Miniaturisation of the IVTT protein array was achieved by robotic gridding of individual IVTT proteins onto a glass surface pre-bound with streptavidin. The glass "chips" with streptavidin attached covalently to one surface were purchased from Radius Inc (Medfield, MA, USA). Glass chips were 5 5 mm x 25mm. Using the gridding robot (Radius Inc, Medfield, MA, USA), 0.5µl of each of 96 IVTT reactions was applied to the dry surface of the chip in a low density array of 8 by 12. In further experiments, double density arrays of 192 individual proteins per glass chip were produced. In subsequent experiments very high density arrays suitable for high throughput screening were assembled. Following gridding, the chip surface was washed using phosphate buffered saline (PBS) and slides stored at 4°C in PBS containing 3% (w/v) BSA and 0.02% sodium azide.

The IVTT protein chip arrays were treated with tissue lysates and probed with anti-phosphoprotein antibodies broadly as given in example 2. Protein chip arrays were washed extensively by immersion in tris buffered saline (TBS) prior to incubation with brain tissue lysates. Excess washing solution was shaken from the slide before 15µl of brain tissue lysate was applied directly to the surface over the area encompassing the protein array. A glass coverslip was placed over the tissue lysate solution serving to spread the lysate over the whole area of the array and being held by capillary effect stop significant solution loss during the subsequent incubation. The incubation was conducted for 30 minutes at 37°C with the glass chip placed in a humidified container. Following incubation, the coverslip was removed and the slide washed by immersion in wash buffer. The glass chip was incubated with one or combinations of anti- phosphoprotein antibodies diluted as per example 2. Antibody solution was applied in small volume (10-20µl) to the protein array, and held in place under a coverslip as previously. Detection of bound antibody was by use of chromogenic substrates yielding insoluble precipitate onto the glass slide. Typically, substrate DAB (diaminobenzidine tetrahydrochloride) (Sigma, Poole, UK) was used. Positive signals in low density arrays were identified by visual examination of the array under low-power magnification. For some experiments an enhanced chemiluminescent substrate (Pierce & Warriner Ltd, Chester UK) was used in combination with peroxidase conjugated primary or secondary antibodies. Positive signals were detected using a Molecular Imager FX (BioRad Laboratories Ltd, Herts, UK) phosphor imager system.

Protein-protein binding assays were performed on the IVTT protein glass chip arrays using FITC labelled brain protein and broadly as described in example 5. 10-20µl FITC labelled brain protein preparation was applied directly to the surface of a washed IVTT protein array. A glass coverslip was placed over the labelled tissue lysate solution, and the complete slide incubated at 37°C for 30 minutes in a humidified container. Following incubation, the coverslip was removed and the slide washed by immersion in wash buffer.

In some experiments the bound FITC labelled protein was detected directly by fluorescence microscopy. For microscopy, the protein array was covered with one drop of antifade mountant (Vector Labs, Peterborough, UK) and a conventional coverslip for oil immersion fluorescence microscopy. The microscope was a Nikon type 105 fitted with an FITC compatible filter set for fluorescence microscopy. The position of protein spots giving positive signal was de-coded by reference to three-digit readings on the X-Y axis from the microscope stage under fluorescence illumination. These readings were related to the protein array co-ordinates following examination of an index spot etched on each glass surface prior to robotic gridding. The index spot was identified under conventional illumination to give the X-Y readings for its position relative to the protein array.

Some experiments were conducted using indirect detection of the bound labelled protein. For these, and anti-FITC monoclonal antibody was used, followed by an anti-mouse peroxidase conjugate and colourimetric detection using DAB as the reporter substrate. In this instance, reagents were from Sigma (Poole, UK) and the incubations were conducted using small volumes (10-20µl) of diluted antibodies applied directly to the glass surface and held under coverslips as previously described. Positive signals were identified by visual examination of the array using low-power magnification under conventional illumination. Further experiments also used an enhanced chemiluminescent substrate (Pierce & Warriner Ltd, Chester UK) and imaging using a phosphor imager system as previously.

### Example 8

### Method for production of a single chain antibody library immobilised onto a solid phase array for reaction with tissue extracts.

An extract of normal human brain, prepared as in example 4, was used to immunise two BalbC mice. 2 doses were given intra-peritoneally with an interval of 4 weeks between them. 3 to 4 days after the 2nd inoculation, the mice were sacrificed and spleens removed by dissection. A 25 cm² tissue culture flask was coated with the brain extract proteins at 25 µg/ml in carbonate/bicarbonate buffer pH 9.0. After incubation for 1 hour at 37°C, the antigen preparation was removed and sterile blocking buffer (2% non-fat dry milk in PBS) added and incubated for 1 hour. After washing 3 times with PBS, 25 ml of spleen cell suspension in tissue culture medium was added to the flask and incubated overnight at 37°C in a 5% CO₂ atmosphere. The medium containing the non-adherent spleen cells was removed. RNA preparation was then initiated from the adherent selected cells by adding 1.5 ml of the Extraction buffer from the QuickPrep™ mRNA purification kit (Pharmacia, St Albans, UK) to the flask and swirling over the surface. 3 ml of the Elution buffer was then added and mixed. The flask contents were then transferred to a 15 ml centrifuge and the QuickPrep™ mRNA preparation continued according to the manufacturer's instructions

The Pharmacia Recombinant Phage Antibody System (Pharmacia, Milton Keynes, UK) was used to produce a library of mouse single chain Fvs (ScFv) against human brain. First-strand cDNA was generated from the mRNA using M-MuLV reverse transcriptase and random hexamer primers. Antibody heavy and light chain genes were then amplified using specific heavy and light chain primers complementary to conserved sequences flanking the antibody variable domains. The 340 and 325 base pair products generated for heavy and light chain DNA respectively were separately purified following agarose gel electrophoresis. These were then assembled into a single ScFv construct using a DNA linker-primer mix to give the VH region joined by a (Gly4Ser)3 peptide to the VL region. The assembled ScFv were amplified with primers designed to insert Sfi 1 and Not 1 sites at the 5' and 3' ends respectively, giving an 800 bp product. This fragment was purified, sequentially digested with SfiI and NotI, and repurified.

For production of an *in vitro* transcribed and translated ScFv library, the vector pBluescript SK+ was modified by the insertion of the linker between the HindIII and *Eco* RI sites in the multiple cloning site region:

The amplified ScFv were cloned between the SfiI and NotI sites of this vector such that they were inserted in the correct orientation for transcription from the T7 promoter. Alternatively, a human naive phage antibody library (Nissin, MRC) was used. Phagemid DNA was prepared and cut sequentially with SfiI and NotI. The ScFv fragments were purified and cloned into the modified pBluescript SK+ vector described above.

For use as a solid phase array, individual pBluescript clones were picked and grown in multiwell dishes as per example 3. DNA preparation, IVTT and array of biotinylated proteins was as per example 3.

For use as a phage antibody library, the Pharmacia protocol was followed. The assembled ScFv were amplified with primers designed to insert SfiI and NotI sites at the 5' and 3' ends respectively, giving an 800 bp product. This fragment was purified, sequentially digested with Sfi 1 and Not 1, repurified and ligated into SfiI and NotI cut pCANTAB 5 phagemid vector. PCANTAB 5 contains the gene encoding the Phage Gene 3 protein (g3p) and the ScFv is inserted adjacent to the g3 signal sequence such that it will be expressed as a g3p fusion protein. Competent *E.coli* TG1 cells were transformed with the pCantab 5/ScFv phagemid then subsequently infected with the M13KO7 helper phage. The resulting recombinant phage contained DNA encoding the ScFv genes and displayed one or more copies of recombinant antibody as fusion proteins at their tips.

Phage-displayed ScFv that bind to human brain antigens were then selected or enriched by panning. Briefly brain antigen preparation was coated onto wells of 96-well microtitre plates. After blocking with 2% non-fat dry milk in PBS, the phage preparation was applied and incubated for 1 hour. After washing extensively with PBS, wells containing brain antigen reactive recombinant phage were detected with horse radish peroxidase conjugated anti-M13 antibody and revealed with o-phenylene diamine chromogenic substrate.

### Example 9

### IVTT Display library gridded and treated with labelled tissue protein.

A single chain antibody IVTT display library was translated *in vitro* and arrayed onto streptavidin surface as per example 3. The single chain antibody array was reacted with FITC labelled brain proteins as prepared in example 5. Reaction procedure and detection of protein:protein binding was as per example 5. Patterns of positive signals from replicate IVTT arrays treated with different tissue proteins were compared. For identification of individual proteins at specific loci on the arrays, the corresponding single chain antibody gene was recovered from the master plate of library cDNAs. Single chain antibody genes of interest were then resubjected to larger scale IVTT and used either to retrieve and identify binding partners from an IVTT protein library as described in example 6 or to retrieve and identify (by 2D gels) natural tissue protein as described in example 1.

### Example 10

### Use of DNA chips to form a ribosome display

In order to form a polysome display library for subsequent immobilisation onto a DNA chip, the library would be formed essentially as described in example 1 except that one of the primers used to clone the cDNAs into the IVTT vectors would comprise a mixed primer with a tract of variable sequence within the transcribed sequence designed to anneal to the DNA molecules on the chip. Alternatively, the plasmid pool from the display library would be digested with a unique restriction enzyme within the transcribed sequence and a mixed synthetic oligonucleotide cloned in. Thus the library would be constructed such that each mRNA molecule produced would contain a unique sequence tag; this would be at least 15 nucleotides, preferably about 20 nucleotides.

The experiments to test the DNA chip protein array method were conducted using a pair of modified single chain antibody (scAbs) genes. Two modified scAbs were prepared consisting ofN-terminal epitope tags, the heavy chain variable region (VH), a 14 amino acid linker (EGKSSGSGSESKVD), the light chain variable region (VL), fused to cDNA for the human κ constant region. The human κ constant region sequence, which included a poly-Histidine tag at the 3' end, was modified to delete the stop codons. The human κ constant region is included to act as a spacer to allow correct folding of nascent single chain Fv while still attached to a ribosome complex in an *in vitro* translation system.

These constructs were cloned into the vector pET 5c (Rosenberg AH et al., Gene, 56:125-135, 1987) which provides a T7 promoter followed by the ribosome binding site from T7 gene 10. The scAb constructs were inserted into the vector at an NdeI site such that the sequence encoding the epitope tag followed the first Atg of T7 gene 10. The first construct consisted of a scAb against Pseudomonas aeruginosa (Molloy P. et al. Journal of applied Bacteriology, 78:359-365, 1995) with the FLAG epitope (MDYKDDDK) (Knappik A and Pluckthun A, BioTechniques, 17: 754-761, 1994) added at the N terminus. The second consisted a scAb constructed from the antibody 340 (Durrant LG et al. Prenatal Diagnosis, 14:131-140, 1994) with a poly-Histidine tag at the N terminus.

These plasmids served as parents for subsequent derivatives. The anti*-Pseudomonas aeruginosa* (α-Ps) scAb and the 340 scAb were constructed as follows. DNA for the α-Ps scAb in the vector pPM1His (Molloy P et al., ibid) was amplified with the primers RD 5' FLAG: 5'gcggatcccatatggactacaaagacgatgacgacaaacaggtgcagctgcag3' (Genosys Biotechnologies Europe Ltd, Cambridge, UK) and RD 3': 5'gcgaattcgtggtggtggtggtggtgtgactctcc3' (Genosys) which introduced the 5' FLAG epitope sequence and removed the 3' stop codon respectively. The reaction mixture included 0.1 µg template DNA, 2.6 units of Expand™ High Fidelity PCR enzyme mix (Boehringer Mannheim, Lewes, UK.), Expand HF buffer (Boehringer Mannheim), 1.5 mM MgCl₂, 200 µM deoxynucleotide triphosphates (dNTPs) (Life Technologies, Paisley, UK) and 25 pmoles of each primer. Cycles were 96°C 5 minutes, followed by [95°C 1 minute, 50°C 1 minute, 72°C 1 minute] times 5, [95°C 45 seconds, 50°C 1 minute, 72°C 1 minute 30 seconds] times 8, [95°C 45 seconds, 50°C 1 minute, 72°C 2 minutes] times 5, finishing with 72°C 5 minutes. The 1123 bp product obtained was cut with BamHI and EcoRI and cloned into the vector pUC19 (Boehringer Mannheim). The DNA sequence was confirmed, using the Thermo Sequenase radiolabeled terminator cycle sequencing kit with [³³P] dideoxy nucleotides (Amersham Life Science, Amersham, UK). The construct was cloned into pET5c vector (Promega UK Ltd, Southampton, UK.) as a NdeI to EcoRI fragment (see *Molecular Cloning, A Laboratory Manual* eds. Sambrook J, Fritsch EF, Maniatis T. Cold Spring Harbor Laboratory Press 1989, New York, USA). Plasmid DNA was prepared using Wizard® Plus SV Minipreps DNA purification System (Promega UK Ltd), or for larger scale, Qiagen Plasmid Midi Kit (Qiagen Ltd, Crawley, UK.). The new plasmid generated was named pET5c FLAG-αPs scAb.

The 340 scAb was produced by substitution the VH and VK of the 340 antibody in place of the α-Ps VH and VK in ppM1His. The 340 VH was amplified with the primers 5'cagctgcaggagtctgggggaggcttag3' (Genosys) and 5'tcagtagacggtgaccgaggttccttgaccccagta3' (Genosys). The reaction mixture included 0.1 µg template DNA, 2.6 units of Expand™ High Fidelity PCR enzyme mix, Expand HF buffer, 1.5 mM MgCl2, 200 µM dNTPs and 25 pmoles of each primer. Cycles were 96°C 5 minutes, followed by [95°C 1 minute, 50°C 1 minute, 72°C 1 minute] times 5, [95°C 45 seconds, 50°C 1 minute, 72°C 1 minute 30 seconds] times 8, [95°C 45 seconds, 50°C 1 minute, 72°C 2 minutes] times 5, finishing with 72°C 5 minutes. The 357 bp product was cut with PstI and BstEII and cloned into PstI and BstEII cut pPM1His (see *Molecular Cloning, A Laboratory Manual*, ibid). Similarly, the 340 VK was amplified with the primers 5'gtgacattgagctcacacagtctcct3' and 5'cagcccgttttatctcgagcttggtccg3' (Genosys). The 339 bp product was cut with SstI and XhoI and cloned into SstI and XhoI cut modified pPM1His (produced above). The DNA sequence was confirmed, using the Thermo Sequenase radiolabeled terminator cycle sequencing kit with [³³P] dideoxy nucleotides (Amersham). DNA for the 340 scAb in the vector pPM1His was amplified with the primers RD 5' HIS: 5'gcggatcccatatgcaccatcatcaccatcaccaggtgcagctgcag3' (Genosys) and RD 3' (given above) which introduced the 6 histidine residues at the 5' end and removed the 3' stop codon respectively. Reagents and conditions for amplification were exactly as for the α-Ps construct. The 1114 bp product obtained was cut with BamHI and EcoRI and cloned into the vector pUC 19 (see *Molecular Cloning, A Laboratory Manual,* ibid). The DNA sequence was confirmed as before and the construct was cloned into pET5c vector as a NdeI to EcoRI fragment to generate the plasmid pEt5c HIS 340 scAb.

Model genes were modified to contain a nucleic acid "capture domain" sequence. Synthetic oligonucleotides used were as follows;

The capture domain sequence was derived from the SFFV friend erythroleukeamia virus and has previously been shown to have no complementary sequence in mammalian cDNA databases [Tavitian et al (1998), *Nature Medicine* 4: 467-471].
The capture domain was inserted either into the 5' UTR region of the model genes described above, or fused into the 3' region of the said genes. Constructs were designated CAP5 or CAP3 variants respectively. Insertion of the CAP5 and CAP3 domains was achieved by PCR using primers designed to be universal for each of the available model genes. For CAP5 insertion, two serial PCRs were conducted using primers CAP5 inner and RD3, followed by a second PCR with primers CAP5 outer and RD3. The resulting product was purified and used directly in an *in vitro* transcription reaction. Insertion of the CAP3 domain proceeded according to the same scheme, however primer CAP3 innner was used in the primary PCR with primer T7loop, followed by a secondary PCR using primers CAP3 outer and T7loop. The purified product was used directly in an *in vitro* transcription reaction. Transcription reactions were carried out using T7 RNA polymerase and the RiboMAX system (Promega, Southampton UK) using conditions recommended by the supplier.

RNA was hybridised to a synthetic DNA oligonucleotide covalently attached to the surface of a microtitre plate. The capture oligonucleotide was of complementary sequence to the capture domain within the RNA. In some experiments the capture oligonucleotide was attached via a 5'-amino-linker moiety, and in other experiments attachment was via the 3' end of the molecule. Microtitre plates with attached oligonucleotides were supplied under contract by GenoSys Biotechnologies Europe Ltd (Cambridge, UK). The hybridisation reaction was conducted for 60 minutes at 55°C in a reaction buffer containing tetramethylammonium chloride as described by Maskos and Southern (Maskos U & Southern E.M. (1992) *Nucleic Acids Res.* 20: 1675-1678). Post hybridisation washes were conducted to high stringency using dilutions of an SSPE buffer containing 0.1% (v/v) SDS (1x SSPE buffer = 180mM NaCl, 10mM sodium phosphate pH 7.7, 1mM ethylenediaminetetra acetic acid).

Following the final wash, *in vitro* translation reaction was initiated by addition of 25µl rabbit reticulocyte lysate (Promega) supplemented with a complete amino acid mixture. In some experiments a methionine minus amino acid mix was used, in this case ³⁵S-methionine (Amersham) was added. The translation reaction was conducted at 30°C for 60 minutes then placed on ice. Wells were washed using ice cold PBS containing 1% (w/v) BSA. Translation products were detected using antibodies for either the Flag or the his6 epitope engineered into each of the model gene constructs. Antibodies were added to the washed wells diluted in PBS. Incubations were for 60 minutes at 4°C with gentle mixing. A secondary reagent (anti-mouse-HRP conjugate), was added at the recommended dilution in PBS and incubated for a further 30 minutes at 4°C. Wells were washed three times using 200µl PBS before colour development with the chromogenic substrate. Plates were read at 492nm. Where ³⁵S-methionine was included in the reaction mix, translation products were detected by scintillation counting. The results indicated successful immobilisation of the correct protein by virtue of attachment to its associated mRNA in turn annealed to the corresponding immobilised DNA by virtue of the complementary region of sequence.

## Claims

1. A method of screening proteins or polypeptides to identify a biologically active protein or polypeptide of interest comprising the steps:
(i) forming a gene library
(ii) expressing from said polynucleotide library, wherein the polynucleotides are distributed into an array, synthetic proteins or polypeptides by in vitro transcription and translation (IVTT), thus generating an array of individually identifiable proteins or polypeptides,
(iii) immobilising the proteins or polypeptides in said array obtained by step ii,
(iv) contacting said immobilised proteins or polypeptides in said array of step iii with a sample,
(v) selecting from the array of step iv individual proteins or polypeptides for which interactions with said sample have been detected, and
(vi) identifying the protein or polypeptide of interest selected according to step v by sequencing the corresponding polynucleotide from said original gene library that encodes said selected protein or polypeptide.

2. A method according to claim 1, wherein said gene library is a DNA or RNA library.

3. A method of claim 1 or 2, wherein the immobilisation is achieved onto solid phases.

4. A method of claim 3, wherein the solid phase is a microtitre plate, wherein the proteins or polypeptides are immobilised at specific loci on the surface of the plate.

5. A method according to any of the claims 1 to 4, wherein the interactions with the sample are protein-protein binding interactions.

6. A method of claim 5, wherein the protein-protein interactions result in enzymatic modification of the synthetic proteins or polypeptides in the array.

7. A method according to any of the claims 1 to 6, wherein the sample is a specific tissue molecule, a specific cell molecule, a cell extract or a tissue extract.

8. A method of claim 7, wherein the sample includes a control sample which is not affected by a specific disease, healthcare or drug treatment status, and a diseased sample which is affected by a disease, healthcare or drug treatment status.

9. A method according to any of the claims 1 to 8, wherein the gene library is a cDNA library comprising polynucleotides that encode single chain antibodies (SCA).

10. A method for high-throughput analysing proteins or polypeptides in normal and diseased tissues or cells in order to detect differences between normal and disease proteins, the method comprising the steps specified in any of the claims 1 to 7, wherein a control sample is used which is not affected by a specific disease, healthcare or drug treatment status, and a sample is used which is affected by a disease, healthcare or drug treatment status.

## Patentansprüche

1. Verfahren zum Screenen von Proteinen oder Polypeptiden zur Identifizierung eines biologisch aktiven Proteins oder Polypeptids von Interesse, umfassend die Schritte:
(i) Herstellen einer Genbank,
(ii) Exprimieren synthetischer Proteine oder Polypeptide aus der Polynukleotid-Bank, wobei die Polynukleotide in einer Anordnung verteilt sind, durch eine In-vitro-Transkription und Translation (IVTT), wodurch eine Anordnung von individuell identifizierbaren Proteinen oder Polypeptiden erzeugt werden.
(iii) Immobilisieren der Proteine oder Polypeptide in der durch Schritt ii erhaltenen Anordnung,
(iv) Zusammenbringen der immobilisierten Proteine oder Polypeptide in der Anordnung von Schritt iii mit einer Probe,
(v) Auswählen einzelner Proteine oder Polypeptide, für die Wechselwirkungen mit der Probe nachgewiesen wurden, aus der Anordnung von Schritt iv, und
(vi) Identifizieren des aus Schritt v ausgewählten Proteins oder Polypeptids von Interesse, durch Sequenzieren des entsprechenden Polynukleotids aus der ursprünglichen Genbank, die das ausgewählte Protein oder Polypeptid codieren.

2. Verfahren nach Anspruch 1, wobei die Genbank eine DNA- oder RNA-Bank ist.

3. Verfahren nach Anspruch 1 oder 2, wobei die Immobilisation auf festen Phasen erzielt wird.

4. Verfahren nach Anspruch 3, wobei die feste Phase eine Mikrotiterplatte ist, wobei die Proteine oder Polypeptide an bestimmten Stellen auf der Oberfläche der Platte an bestimmten Stellen immobilisiert sind.

5. Verfahren nach einem der Ansprüche der Ansprüche 1 bis 4, wobei die Wechselwirkungen mit den Proben Protein-Protein-Bindungs-Wechselwirkungen sind.

6. Verfahren nach Anspruch 5, wobei die Protein-Protein-Wechselwirkungen zu einer enzymatischen Modifikation der synthetischen Proteine oder Polypeptide in der Anordnung führen..

7. Verfahren nach einem der Ansprüche 1 bis 6, wobei die Probe ein spezifisches Gewebemolekül, ein spezifisches Zellmolekül, ein Zellextrakt oder ein Gewebeextrakt ist.

8. Verfahren nach Anspruch 7, wobei die Probe eine Kontrollprobe, die nicht von einem spezifischen Erkrankungs-, Gesundheitsfiirsorge- oder Medikamentenbehandlungsstatus betroffen ist und eine erkrankte Probe umfasst, die von einem spezifischen Erkrankungs-, Gesundheitsfürsorge- oder Medikamentenbehandlungsstatus betroffen ist.

9. Verfahren nach einem der Ansprüche 1 bis 8, wobei die Genbank eine cDNA-Bank ist, die Polynukleotide umfasst, welche einzelkettige Antikörper (SCA) codieren.

10. Verfahren für Hochdurchsatz-Analyse-Proteine- oder -Polypeptide in normalen und erkrankten Geweben oder Zellen, um die Unterschiede zwischen normalen und erkrankten Proteinen nachzuweisen, wobei das Verfahren die Schritte nach einem der Ansprüche 1 bis 7 umfasst, wobei eine Kontrollprobe verwendet wird, die nicht von einem spezifischen Erkrankungs-, Gesundheitsfürsorge- oder Medikamentenbehandlungsstatus betroffen ist, und eine Probe verwendet wird, die von einem spezifischen Erkrankungs-, Gesundheitsfürsorge- oder Medikamentenbehandlungsstatus betroffen ist.

## Revendications

1. Procédé de criblage de protéines ou de polypeptides afin d'identifier une protéine ou un polypeptide biologiquement actif digne d'intérêt, comprenant les étapes de :
(i) formation d'une bibliothèque de gènes,
(ii) expression, à partir de ladite bibliothèque de polynucléotides où les polynucléotides sont distribués selon un réseau, de protéines ou de polypeptides synthétiques au moyen d'une transcription et d'une traduction in vitro (IVTT), d'où ainsi la génération d'un réseau de protéines ou de polypeptides identifiables individuellement,
(iii) immobilisation des protéines ou des polypeptides dans ledit réseau qui est obtenu au moyen de l'étape ii,
(iv) mise en contact desdites protéines ou desdits polypeptides immobilisés dans ledit réseau de l'étape iii avec un échantillon,
(v) sélection, à partir du réseau de l'étape iv, de protéines ou de polypeptides individuels pour lesquels des interactions avec ledit échantillon ont été détectées, et
(vi) identification de la protéine ou du polypeptide digne d'intérêt sélectionné conformément à l'étape v en séquencant le polynucléotide correspondant à partir de ladite bibliothèque de gènes originale qui code ladite protéine ou ledit polypeptide sélectionné.

2. Procédé selon la revendication 1, dans lequel ladite bibliothèque de gènes est une bibliothèque d'ADN ou d'ARN.

3. Procédé selon la revendication 1 ou 2, dans lequel l'immobilisation est réalisée sur des phases solides.

4. Procédé selon la revendication 3, dans lequel la phase solide est une plaquette microtitre, dans lequel les protéines ou les polypeptides sont immobilisés en des lieux spécifiques sur la surface de la plaquette.

5. Procédé selon l'une quelconque des revendications 1 à 4, dans lequel les interactions avec l'échantillon sont des interactions de liaison protéine-protéine.

6. Procédé selon la revendication 5, dans lequel les interactions protéine-protéine aboutissent à une modification enzymatique des protéines ou des polypeptides synthétiques dans le réseau.

7. Procédé selon l'une quelconque des revendications 1 à 6, dans lequel l'échantillon est une molécule de tissu spécifique, une molécule de cellule spécifique, un extrait de cellule ou un extrait de tissu.

8. Procédé selon la revendication 7, dans lequel l'échantillon inclut un échantillon de contrôle qui n'est pas affecté par un état spécifique de maladie, de soins de santé ou de traitement par médicament, et un échantillon malade qui est affecté par un état de maladie, de soins de santé ou de traitement par médicament.

9. Procédé selon l'une quelconque des revendications 1 à 8, dans lequel la bibliothèque de gènes est une bibliothèque de cADN qui comprend des polynucléotides qui codent des anticorps en chaîne simple (SCA).

10. Procédé pour analyser selon un débit élevé des protéines ou des polypeptides dans des tissus ou des cellules normales et malades afin de détecter des différences entre des protéines normales et malades, le procédé comprenant les étapes spécifiées selon l'une quelconque des revendications 1 à 7, dans lequel un échantillon de contrôle est utilisé, lequel n'est pas affecté par un état spécifique de maladie, de soins de santé ou de traitement par médicament, et un échantillon est utilisé, lequel est affecté par un état de maladie, de soins de santé ou de traitement par médicament.


### SEQUENCE LISTING

(1) GENERAL INFORMATION:
   (i) APPLICANT:
      (A) NAME: Biovation Limited
      (B) STREET: Investment House, 6 Union Row
      (C) CITY: Aberdeen
      (D) STATE: Scotland
      (E) COUNTRY: United Kingdom
      (F) POSTAL CODE (ZIP): AB10 1DQ

      (A) NAME: CARR, Francis J
      (B) STREET: Birchlea, The Holdings
      (C) CITY: Balmedie
      (D) STATE: Aberdeenshire
      (E) COUNTRY: United Kingdom
      (F) POSTAL CODE (ZIP): AB23 8XU

      (A) NAME: CARTER, Graham
      (B) STREET: Longhills Cottage
      (C) CITY: By New Machar
      (D) STATE: Aberdeenshire
      (E) COUNTRY: United Kingdom
      (F) POSTAL CODE (ZIP): AB21 7XB

      (A) NAME: HAMILTON, Anita
      (B) STREET: 130 Kings Gate
      (C) CITY: Aberdeen
      (D) STATE: Scotland
      (E) COUNTRY: United Kingdom
      (F) POSTAL CODE (ZIP): AB15 4EQ

      (A) NAME: ADAIR, Fiona
      (B) STREET: Burnside of Aucheldy Tarves
      (C) CITY: Ellon
      (D) STATE: Aberdeenshire
      (E) COUNTRY: United Kingdom
      (F) POSTAL CODE (ZIP): AB41 7NH

      (A) NAME: WILLIAMS, Steven
      (B) STREET: Drumgowan Cottage
      (C) CITY: Auchleven, Insch
      (D) STATE: Aberdeenshire
      (E) COUNTRY: United Kingdom
      (F) POSTAL CODE (ZIP): AB52 6QD
   (ii) TITLE OF INVENTION: METHODS FOR PROTEIN SCREENING
   (iii) NUMBER OF SEQUENCES: 14
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: WO PCT/GB98/02649
(2) INFORMATION FOR SEQ ID NO: 1:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "Kozak translation initiation sequence consensus"
   (xi) SEQUENCE DESCRIPTION: SEQ ID NO: 1:
   GCCGCCACCA TGG                                                    13
(2) INFORMATION FOR SEQ ID NO: 2:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 2:
   AGCTTGGCCC AGCCGGCCAT GGCCCAGGTC CAACTGCAGG AGCTCGAGAT CAAACGGGCG    60
   GCCGCG                                                               66
(2) INFORMATION FOR SEQ ID NO: 3:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 3:
   Glu Gly Lys Ser Ser Gly Ser Gly Ser Glu Ser Lys Val Asp
   1               5                   10
(2) INFORMATION FOR SEQ ID NO: 4:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 4:
   CAGCTGCAGG AGTCTGGGGG AGGCTTAG                                        28
(2) INFORMATION FOR SEQ ID NO: 5:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 5:
   TCAGTAGACG GTGACCGAGG TTCCTTGACC CCAGTA                                36
(2) INFORMATION FOR SEQ ID NO: 6:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 6:
   GTGACATTGA GCTCACACAG TCTCCT                                          26
(2) INFORMATION FOR SEQ ID NO: 7:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 7:
   CAGCCCGTTT TATCTCGAGC TTGGTCCG 28
(2) INFORMATION FOR SEQ ID NO: 8:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 8:
   GCGGATCCCA TATGCACCAT CATCACCATC ACCAGGTGCA GCTGCAG 47
(2) INFORMATION FOR SEQ ID NO: 9:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 9:
   AGAATACAGG GTCCAAATAG AATCCAGGGT 30
(2) INFORMATION FOR SEQ ID NO: 10:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 10:
   CTACCTATAA AAATAGGCGT ATCACGAGGC CCTTTCGTCT TCAATAATTC 50
(2) INFORMATION FOR SEQ ID NO: 11:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 11:
   AGCGAATTCA CCCTGGATTC TATTTGGACC CTGTATTCTA CCTATAAAAA TAGG 54
(2) INFORMATION FOR SEQ ID NO: 12:
   (i) SEQUENCE CHARACTERISTICS:
      (A) LENGTH: 61 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 12:
   GGTTTCCCTC TAGAATACAG GGTCCAAATA GAATCCAGGG TAAGAAGGAG ATATACATAT 60
   G 61
(2) INFORMATION FOR SEQ ID NO: 13:
   (i) SEQUENCE CHARACTERISTICS:
      (A) LENGTH: 67 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 13:
   ATATATATGT CGACGAAATT AATACGACTC ACTATAGGGA GACCACAACG GTTTCCCTCT 60
   AGAATAC 67
(2) INFORMATION FOR SEQ ID NO: 14:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 14:
   ATATATATGT CGACGAAATT AATACGACTC ACTATAGGGA GACCACAACG 50